(19) Europäisches Patentamt
European Patent Office
Office européen des brevets

(11) EP 0 415 456 B1

(12) **EUROPEAN PATENT SPECIFICATION**

(45) Date of publication and mention of the grant of the patent:
**26.06.1996 Bulletin 1996/26**

(51) Int Cl.[6]: **C07D 473/06**, C07D 473/08, C07D 473/20, C07D 473/22, A61K 31/52

(21) Application number: **90116791.6**

(22) Date of filing: **31.08.1990**

# (54) Xanthine compounds

Xanthinverbindungen

Dérivés de xanthine

(84) Designated Contracting States:
**AT BE CH DE DK ES FR GB GR IT LI LU NL SE**

(30) Priority: **01.09.1989 JP 226642/89**

(43) Date of publication of application:
**06.03.1991 Bulletin 1991/10**

(73) Proprietor: **KYOWA HAKKO KOGYO CO., LTD.**
**Chiyoda-ku, Tokyo (JP)**

(72) Inventors:
- **Suzuki, Fumio**
  **Mishima-shi, Shizuoka-ken (JP)**
- **Shimada, Junichi**
  **Sunto-gun, Shizuoka-ken (JP)**
- **Ishii, Akio**
  **Sunto-gun, Shizuoka-ken (JP)**
- **Ohno, Tetsuji**
  **Sunto-gun, Shizuoka-ken (JP)**
- **Karasawa, Akira**
  **Huntingdon Valley, PA19006 (US)**
- **Kubo, Kazuhiro**
  **Tagata-gun, Shizuoka-ken (JP)**
- **Nonaka, Hiromi**
  **Sunto-gun, Shizuoka-ken (JP)**

(74) Representative: **VOSSIUS & PARTNER**
**Postfach 86 07 67**
**81634 München (DE)**

(56) References cited:
**EP-A- 0 256 692**
**EP-A- 0 267 607**
**EP-A- 0 374 808**
**DE-B- 1 245 969**
**GB-A- 1 201 997**

- **CHEMICAL ABSTRACTS, vol. 63, no. 13, 20th December 1965, page 18120, column 2, abstract no. 18120d, Columbus, Ohio, US; & DD-A- 31 772**
- **TETRAHEDRON LETTERS, vol. 27, no. 52, 1986, pages 6337-6338, Pergamon Journals Ltd, GB; E. CASTAGNINO et al.: "Decarboxylative radical addition onto protonated heteroaromatic systems including purine bases"**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 415 456 B1

## Description

The present invention relates to novel xanthine compounds having a diuretic effect, a renal-protecting effect and a bronchodilatory effect.

Heretofore, theophylline, i.e., 1,3-dimethylxanthine has been known as a diuretic or a vasodilator [The Merck Index, 10th edition, 9110 (1983)].

Xanthine compounds carrying, at the 8-position thereof, substituents such as alkyl, alicyclic alkyl, aralkyl or aryl groups have a diuretic effect, as disclosed in DD-A-31,772 [Chem. Abst., 63, 18120d (1965)] and DE-A-1,245,969 [Chem. Abst., 67, 90994n (1967)].

In relation to the compounds of the present invention, 8-(1-adamantyl)-1,3,7-trimethylxanthine is described in Tetrahedron Lett., 27, 6337 (1986). However, nothing is mentioned on its pharmacological effect.

The object of the present invention is to provide novel xanthine compounds exhibiting a strong diuretic and renal-protecting effect, based on the finding that xanthine compounds which are adenosine receptor antagonists, particularly those having an activity of selectively antagonizing adenosine $A_1$ receptors have a strong diuretic and renal-protecting effect.

The present invention relates to xanthine compounds represented by the following formula (I):

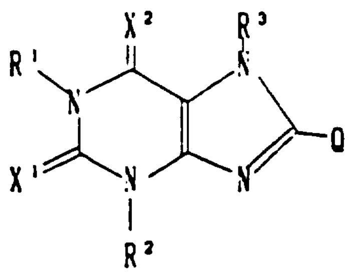

(I)

wherein each of $R^1$, $R^2$ and $R^3$ independently represents a hydrogen atom or a lower alkyl group having 1 to 6 carbon atoms; each of $X^1$ and $X^2$ independently represents an oxygen or sulfur atom; and Q represents:

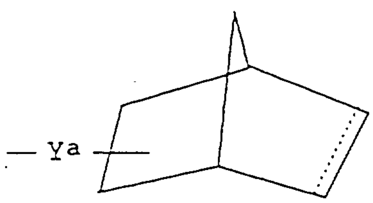 , 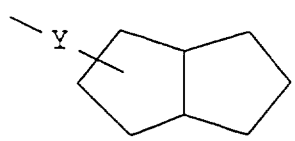 , 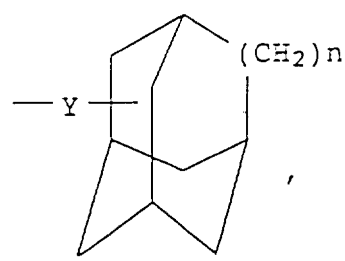 ,

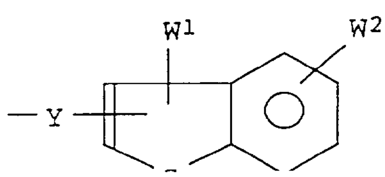 or 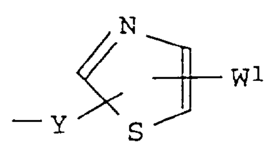

wherein ------- represents a single bond or a double bond;
$Y^a$ represents an alkylene group having 1 to 4 carbon atoms,
Y represents a single bond or an alkylene group having 1 to 4 carbon atoms, n represents 0 or 1, each of $W^1$ and $W^2$ independently represents a hydrogen atom, a lower alkyl having 1 to 6 carbon atoms or amino group, Z represents -$CH_2$-, -O-, -S- or -NH- residues; provided that when Q is

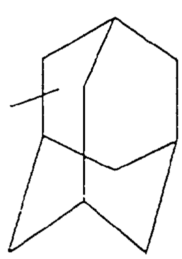 ,

then $R^1$, $R^2$ and
$R^3$ are not simultaneously a methyl group;
or a physiologically acceptable salt thereof.

In the definition of the respective groups in the formula (I), the lower alkyl group includes straight or branched alkyl groups having 1 to 6 carbon atoms for example, methyl, ethyl, propyl, isopropyl, butyl, isobutyl, sec-butyl, tert-butyl, pentyl, neopentyl or hexyl groups. The alkylene group includes straight or branched alkylene groups having 1 to 4 carbon atoms, for example, methylene, ethylene, trimethylene, tetramethylene, methylmethylene, propyplene or ethylethylene.

The physiologically acceptable salts of Compound (I) include physiologically acceptable acid addition salts, metal salts, ammonium salts, organic amine addition salts or amino acid addition salts.

The physiologically acceptable acid addition salts of Compound (I) include inorganic acid salts such as hydrochloride, sulfate or phosphate, and organic acid salts such as acetate, maleate, fumarate, oxalate or citrate.

The physiologically acceptable metal salts include alkali metal salts such as sodium salts or potassium salt, alkaline earth metal salts such as magnesium salt or calcium salt, and also aluminum and zinc salts.

The physiologically acceptable ammonium salts include salts of ammonium or tetramethylammonium . The physiologically acceptable organic amine addition salts include addition salts of morpholine or piperidine, and the physiologically acceptable amino acid addition salts include addition salts of lysine, glycine or phenylalanine.

A process for producing Compound (I) of the present invention is described below.

Compound (I-1) which is Compound (I) wherein $R^3$ is a hydrogen atom, is produced by the following production steps:

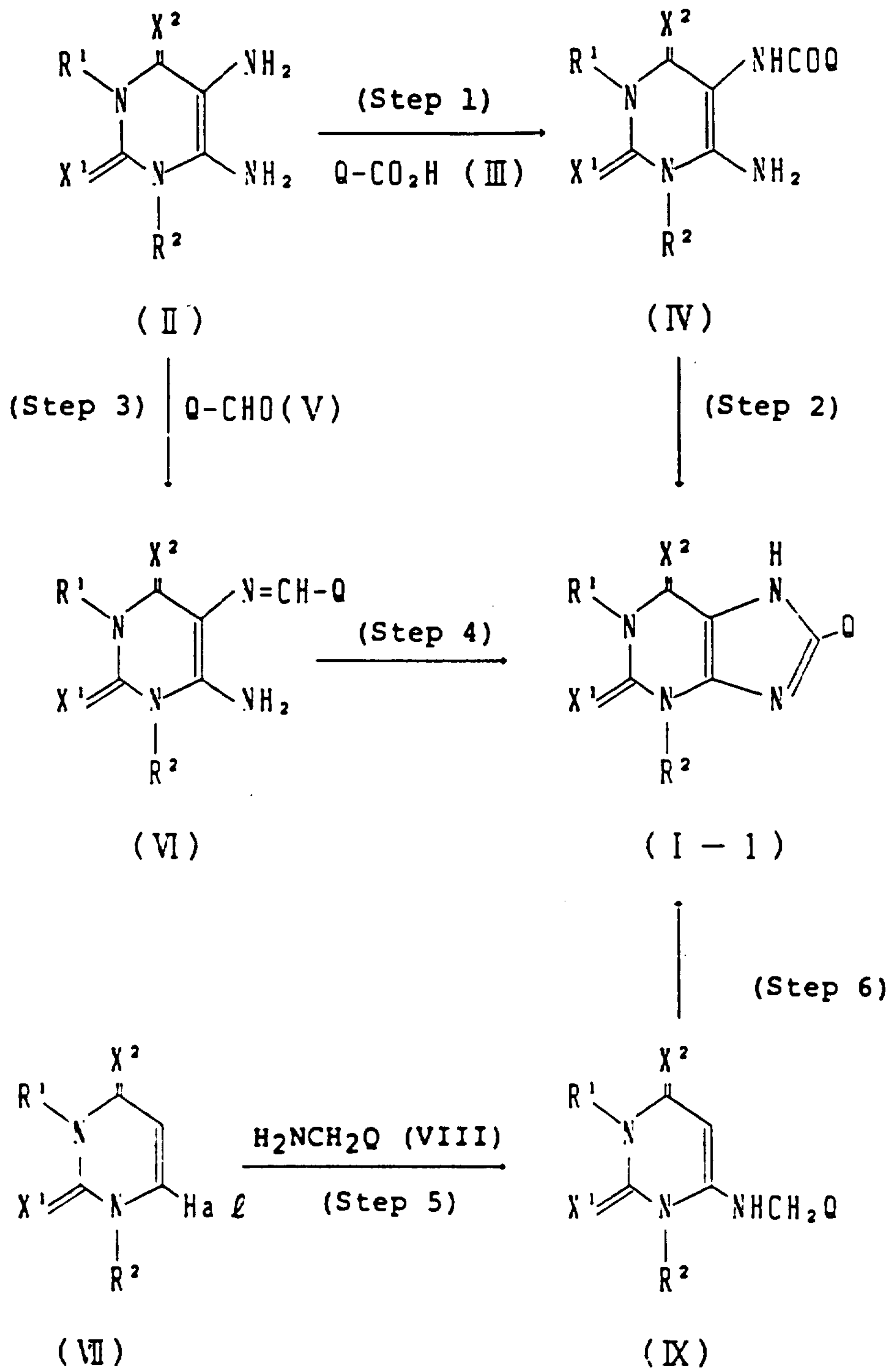


wherein Hal represents halogen atoms such as chlorine, bromine or iodine atoms and $R^1$, $R^2$, $X^1$, $X^2$ and Q have the same meanings as defined above.

<u>Step 1:</u>

A Compound (IV) can be obtained by reacting a uracil derivative (II) obtained according to a well known process [for example, the process disclosed in JP-A-42383/84) with a carboxylic acid (III) or a carboxylic acid reactive derivative.

The carboxylic acid reactive derivative includes acid halides such as acid chlorides or acid bromides, active esters such as p-nitrophenylester or N-oxysuccinimide ester, acid anhydrides commercially available or those formed from carbodiimides such as 1-ethyl-3-(3-dimethylaminopropyl)carbodiimide, diisopropylcarbodiimide or dicyclohexylcarbodiimide or mixed acid anhydrides with monoethyl carbonate or monoisobutyl carbonate and so forth.

The reaction of Compound (II) with Compound (III) is carried out without any solvent at a temperature of 50 to 200°C. In the case of using the carboxylic acid reactive derivative, the reaction can be carried out according to a process usually used in the peptide chemistry. For example, the reaction solvent is properly selected from halogenohydrocarbons such as methylene chloride, chloroform or dichloroethane, ethers such as dioxane or tetrahydrofuran, dimethylformamide and dimethylsulfoxide, and if necessary water is used. The reaction temperature is -80 to 50°C, and the reaction is completed within 0.5 to 24 hours. Sometimes, the reaction may be favorably carried out, if necessary, in the presence of an additive such as 1-hydroxybenzotriazole or a base such as pyridine, triethylamine, dimethylaminopyridine or N-methylmorpholine. Furthermore, the carboxylic acid reactive derivative may be formed in the reaction system and used without isolation.

Step 2:

A desired Compound (I-1) is obtained from Compound (IV) by the reaction in the presence of a base (process A), by treatment with a dehydrating agent (process B), or by heating (process C).

As the preferable base in the process A, alkali metal hydroxides such as sodium hydroxide or potassium hydroxide can be exemplified. As the reaction solvent, water, lower alcohols such as methanol or ethanol, ethers such as dioxane or tetrahydrofuran, dimethylformamide or dimethylsulfoxide can be used alone or in combination. The reaction is carried out at a temperature of from room temperature to 180°C and is usually completed within 10 minutes to 6 hours.

As the dehydrating agent in the process B, thionyl halides such as thionyl chloride and phosphorus oxyhalides such as phosphorus oxychloride can be used, and the reaction is carried out at a temperature of from room temperature to 180°C without any solvent or in an inert solvent, for example, halogenohydrocarbons such as methylene chloride, chloroform or dichloroethane or dimethylformamide or dimethylsulfoxide and is usually completed within 0.5 to 12 hours.

In the case of process C, the Compound (I-1) can be obtained by heating Compound (IV) at a temperature of 50 to 200°C in a polar solvent such as dimethylsulfoxide, dimethylformamide or Dowthermo A (product of Muromachi Kagaku Kogyo Kaisha, Ltd.).

Step 3:

A Schiff base (VI) can be obtained by reacting Compound (II) with an aldehyde (V) in a mixed solvent such as a mixture of acetic acid with a lower alcohol such as methanol or ethanol at a temperature of -20 to 100°C.

Step 4:

The desired Compound (I-1) can be obtained by subjecting Compound (VI) to an oxidative cyclization reaction.

As the appropriate oxidizing agent, oxygen, ferric chloride, cerium $^{IV}$ ammonium nitrate or diethyl azodicarboxylate can be exemplified. The reaction is carried out at from room temperature to 180°C in the presence of the afore-mentioned oxidizing agent and, if necessary, in an inert solvent, for example, a lower alcohol such as methanol or ethanol or a halogenohydrocarbon such as methylene chloride or chloroform or an aromatic hydrocarbon such as toluene, xylene or nitrobenzene.

Step 5:

A Compound (IX) can be obtained by reacting an uracil derivative (VII) obtained according to a well known process, for example, the process described in JP-A-5082/86 with an amine (VIII) in an inert solvent, for example, a lower alcohol such as methanol or ethanol or dimethylformamide or dimethylsulfoxide alone or in combination thereof at a temperature of 50 to 150°C.

Step 6:

A Compound (I-1) can be obtained by reacting a Compound (IX) with a nitrosating agent such as sodium nitrite or isoamyl nitrite under acidic conditions, e.g. with dilute hydrochloric acid in an inert solvent; for example, a lower alcohol such as methanol or ethanol usually at a temperature of from room temperature to the boiling point of the solvent.

Step 7:

A Compound (I-2) which is a Compound (I) wherein $R^3$ is a lower alkyl group can be obtained through the following step:

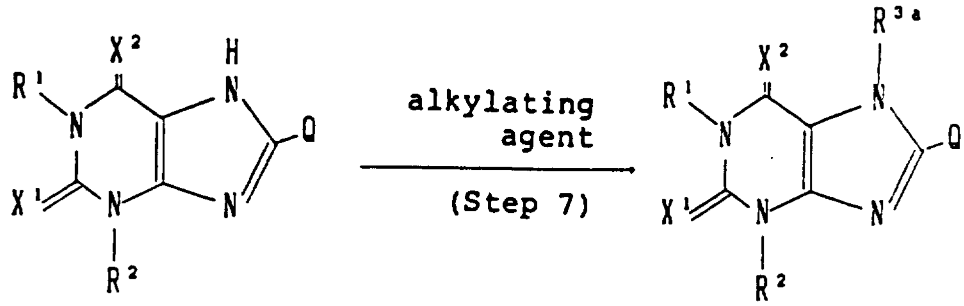


( I – 1 ) ( I – 2 )

wherein $R^1$, $R^2$, $X^1$, $X^2$ and Q have the same meanings as defined above, $R^{3a}$ represents a lower alkyl group within the definition of $R^3$.

The desired compound (I-2) can be obtained by reacting Compound (I-1) obtained in Steps 1 to 6 with an alkylating agent preferably in the presence of a base.

As the alkylating agent, alkyl halides, dialkyl sulfates or diazoalkanes are used.

As the base, an alkali metal carbonate such as sodium carbonate or potassium carbonate, an alkali metal hydride such as sodium hydride and an alkali metal alkoxide such as sodium methoxide or sodium ethoxide are exemplified. The reaction is completed at a temperature of 0 to 180°C usually within 0.5 to 24 hours.

Step 8:

Compound (I-4) which is a Compound (I) wherein $X^2$ is a sulfur atom, can be obtained by the following step:

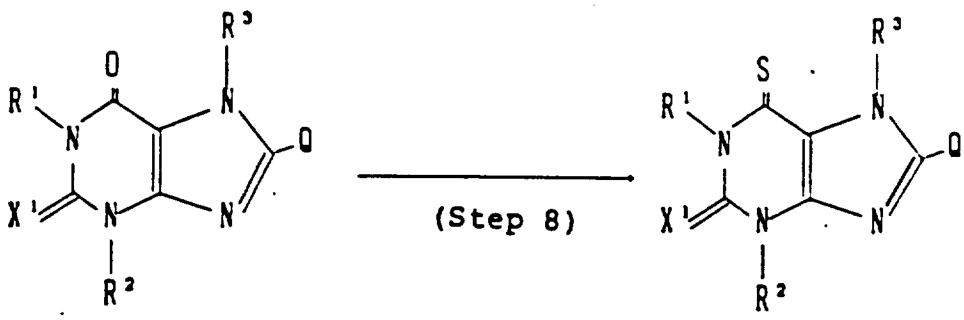


( I – 3 ) ( I – 4 )

wherein $R^1$, $R^2$, $R^3$, $X^1$ and Q have the same meanings as defined above.

The desired Compound (I-4) was prepared by reacting Compound (I-3) which is a Compound (I) wherein $X^2$ is an oxygen atom, with an appropriate thionation reagent, in an inert solvent. As thionation reagent, e.g. phosphorus pentasulfide is mentioned. As solvents, dimethylformamide, pyridine, tetrahydrofuran or dioxane are mentioned, whereby pyridine is preferably used. The reaction is carried out at a temperature of 50 to 180°C for a period of 10 minutes to 36 hours.

The intermediates and the desired compounds obtained according to the aforementioned processes can be isolated and purified by subjecting them to a purification process usually used in the organic synthetic chemistry, for example, filtration, extraction, washing, drying, concentration, recrystallization and/or various chromatographies. The intermediates can be used in the successive reaction without any purification.

Salts of the Compound (I) can be obtained directly by purification when Compound (I) can be obtained in a salt form, or by formation of a salt according to a usual procedure when the Compound (I) is obtained in a free form, and a subsequent purification.

Compound (I) and its physiologically acceptable salts sometimes exist in an adduct form with water or various other solvents, and these adducts are included in the present invention.

Optical isomers may exist with respect to Compound (I), and all the possible stereoisomers and their mixtures are also included in the scope of the present invention.

Specific examples of Compound (I) are shown in Table 1.

Table 1

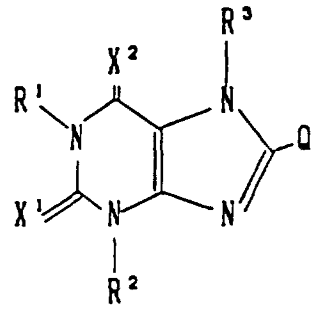

| Compound No. (Example No.) | $R^1$ | $R^2$ | $R^3$ | Q | $X^1$ | $X^2$ |
|---|---|---|---|---|---|---|
| 1 (1) | n-$C_3H_7$ | n-$C_3H_7$ | H | 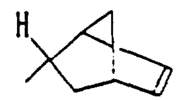 | O | O |
| 2 (1) | n-$C_3H_7$ | n-$C_3H_7$ | H | 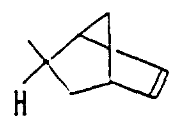 | O | O |
| 3 (2) | n-$C_3H_7$ | n-$C_3H_7$ | H | 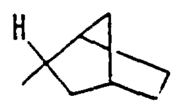 | O | O |
| 4 (2) | n-$C_3H_7$ | n-$C_3H_7$ | H | 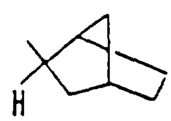 | O | O |
| 5 (3) | n-$C_3H_7$ | n-$C_3H_7$ | H | 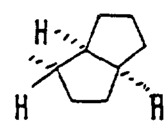 | O | O |
| 6 (3) | n-$C_3H_7$ | n-$C_3H_7$ | H | 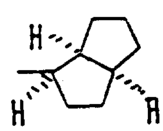 | O | O |
| 7 (4) | n-$C_3H_7$ | n-$C_3H_7$ | H | 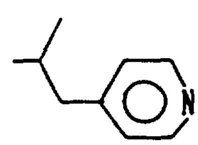 | O | O |

| Compound No. (Example No.) | $R^1$ | $R^2$ | $R^3$ | Q | $X^1$ | $X^2$ |
|---|---|---|---|---|---|---|
| 8 (5) | $n-C_3H_7$ | $n-C_3H_7$ | H | 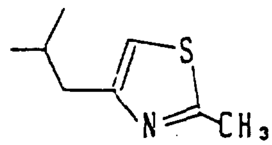 | O | O |
| 9 (6) | $n-C_3H_7$ | $n-C_3H_7$ | H | 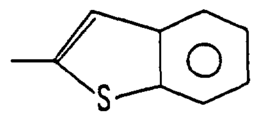 | O | O |
| 10 (7) | $n-C_3H_7$ | $n-C_3H_7$ | H | 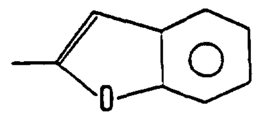 | O | O |
| 11 (8) | $n-C_3H_7$ | $n-C_3H_7$ | H | 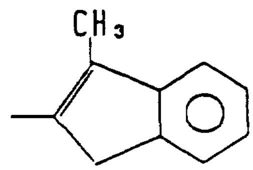 | O | O |
| 12 (9) | $n-C_3H_7$ | $n-C_3H_7$ | H | 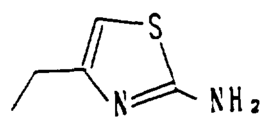 | O | O |
| 13 (29) | $n-C_3H_7$ | $n-C_3H_7$ | H | 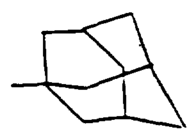 | O | O |
| 14 (10) | $n-C_3H_7$ | $n-C_3H_7$ | H | 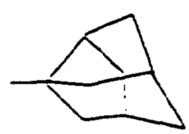 | O | O |
| 15 (11) | $n-C_3H_7$ | $n-C_3H_7$ | H | 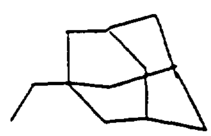 | O | O |
| 16 (12) | $n-C_3H_7$ | $n-C_3H_7$ | H | 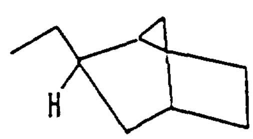 | O | O |

| Compound No. (Example No.) | $R^1$ | $R^2$ | $R^3$ | Q | $X^1$ | $X^2$ |
|---|---|---|---|---|---|---|
| 17 (13) | $n-C_3H_7$ | $n-C_3H_7$ | $CH_3$ | 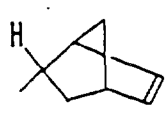 | O | O |
| 18 (14) | $n-C_3H_7$ | $n-C_3H_7$ | $CH_3$ | 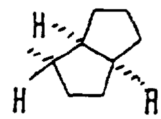 | O | O |
| 19 (30) | $n-C_3H_7$ | $n-C_3H_7$ | $CH_3$ | 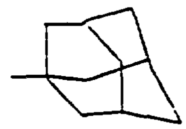 | O | O |
| 20 (15) | $n-C_3H_7$ | $n-C_3H_7$ | $CH_3$ | 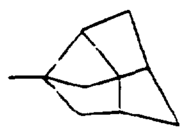 | O | O |
| 21 (16) | $n-C_3H_7$ | $n-C_3H_7$ | $C_2H_5$ | 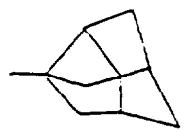 | O | O |
| 22 (17) | $n-C_3H_7$ | $n-C_3H_7$ | $n-C_3H_7$ | 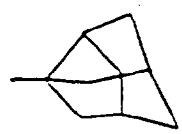 | O | O |
| 23 (18) | H | $n-C_3H_7$ | H | 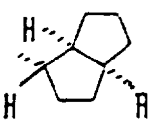 | O | O |
| 24 (19) | $n-C_3H_7$ | $n-C_3H_7$ | H | 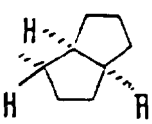 | S | O |
| 25 (20) | H | $n-C_3H_7$ | H | 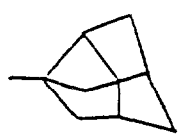 | O | O |

| Compound No. (Example No.) | $R^1$ | $R^2$ | $R^3$ | Q | $X^1$ | $X^2$ |
|---|---|---|---|---|---|---|
| 26 (21) | H | $n-C_3H_7$ | H | 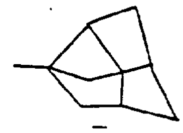 | O | S |
| 27 (22) | $CH_3$ | $CH_3$ | H | 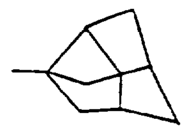 | O | O |
| 28 (23) | $C_2H_5$ | $C_2H_5$ | H | 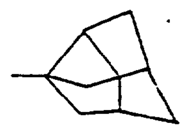 | O | O |
| 29 (24) | $n-C_4H_9$ | $n-C_4H_9$ | H | 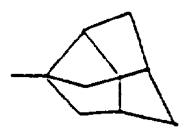 | O | O |
| 30 (25) | $CH_3$ | $iso-C_4H_9$ | H | 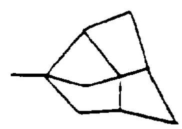 | O | O |
| 31 (26) | $n-C_3H_7$ | $n-C_3H_7$ | H | 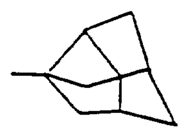 | S | O |
| 32 (27) | $n-C_3H_7$ | $n-C_3H_7$ | H | 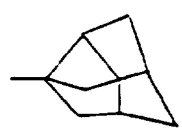 | O | S |
| 33 (28) | $n-C_3H_7$ | $n-C_3H_7$ | H | 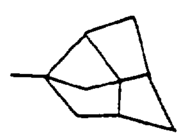 | S | S |

Compound (I) and its physiologically acceptable salts have an activity of selectively antagonizing adenosine $A_1$ receptors, and thus exhibit, a diuretic effect, a renal-protecting effect and a bronchodilatory effect. Compound (I) and its physiologically acceptable salts are useful as a diuretic and renal-protecting agent and bronchodilatory agent.

The pharmacological effects of Compound (I) are explained, referring to Test Examples.

Test Example 1, acute toxicity test

A test compound (300 mg/kg) was orally administered to male dd-strain mice having a body weight of 20± 1 g (3 animals/group). The Minimum lethal dose (MLD) of the compounds was determined by observing whether or not the mice were alive after 7 days of the administration.

With respect to Compound Nos. 1-5, 7-11, 13-18, 20 23, 24 and 31-33, the MLD was more than 300 mg/kg, and

with respect to Compound No. 12, that was 300 mg/kg. This shows that the toxicity of Compound (I) is weak and can be administered safely over a wide range of dosage.

Test Example 2, adenosine receptor binding test

1) Adenosine $A_1$ Receptor Binding

This test was conducted according to the method of Bruns et al. [Proc. Natl. Acad. Sci., 77, 5547 (1980)] with some modifications.

Cerebrum of a guinea pig was suspended into ice cooled 50 mM tris hydroxymethyl aminomethane hydrochloride (Tris HCl) buffer (pH = 7.7), by using Polytron homogenizer (manufactured by Kinematica Co.). The suspension was centrifuged (50,000 x g, 10 minutes), and the precipitate was resuspended by adding the same volume of 50 mM Tris HCl buffer. The suspension was centrifuged under the same conditions, and the precipitate obtained was suspended once again by adding 10 volumes of 50 mM Tris HCl. The tissue suspension was incubated at 37°C for 30 minutes in the presence of 0.02 units/mg tissue of adenosine deaminase (manufactured by Sigma Co.). The resulting tissue suspension was recentrifuged (50,000 x g, 10 minutes), and 50 mM Tris HCl was added to the precipitate to adjust the concentration of tissue to 10 mg (wet weight)/mℓ.

To 1 mℓ of the tissue suspension prepared above were added 50 μℓ of [$^3$H] cyclohexyladenosine [$^3$H-CHA, 27 Ci/mmol, manufactured by New England Nuclear Co.] (final concentration = 1.1 nM) and 50 μℓ of the test compound. The mixture was incubated at 25°C for 90 minutes, and the resulting reaction was stopped by rapid vacuum filtration through a glass fiber filter (GF/C manufactured by Whatman Co.) followed by immediately washing three times with 5 mℓ each of ice cold 50 mM Tris HCl buffer. The filter was transferred to a vial bottle, and a scintillator (EX-H by Wako Pure Chemicals Industries, Ltd.) was added thereto. Its radioactivity was then determined by a scintillation counter (manufactured by Packard Instrument Co.).

The inhibition rate of the test compounds against the binding of $A_1$ acceptor ($^3$H-CHA binding) was calculated from the following equation:

$$\text{Inhibition (\%)} = \left(1 - \frac{[B] - [N]}{[T] - [N]}\right) \times 100$$

[Notes]

1. "B" means the radioactivity of $^3$H-CHA bound in the presence of a test compound at a concentration shown in Table 2.
2. "T" means the radioactivity of $^3$H-CHA bound in the absence of test compounds.
3. "N" means the radioactivity of $^3$H-CHA bound in the presence of 10 μM of $N_6$-(L-2-phenylisopropyl)adenosine (manufactured by Sigma Co.).

The results are shown in Table 2. The inhibition constant (Ki value) shown in the table was calculated from Cheng-Prusoff's equation.

2) Adenosine $A_2$ Acceptor Binding Test

This test was conducted according to the method of Bruns et al. [Mol. Pharmacol., 29, 331 (1986)] with some modifications.

A precipitate was prepared from rat corpus striatum in a similar manner as in 1) above. The precipitate was suspended by adding a 50 mM Tris HCl buffer containing 10 mM magnesium chloride and 0.02 unit/mg (tissue) of adenosine deaminase (manufactured by Sigma Co.) to adjust the concentration of tissue to 5 mg (wet weight)/mℓ.

To 1 mℓ of tissue suspension prepared above were added 50 μℓ of a mixture of N-ethylcarboxamidoadenosine [$^3$H-NECA, 26 Ci/mmol, manufactured by Amersham Co.] (final concentration= 3.8 nM) and cyclopentyladenosine [CPA, manufactured by Sigma Co.] (final concentration= 50 nM), and 50 μℓ of test compound. The mixture was incubated at 25°C within 120 minutes. The resulting mixture was treated in the same manner as in 1) above to determine its radioactivity.

The inhibition rate of the test compound against the binding of $A_2$ receptor ($^3$H-NECA binding) was calculated from the following equation:

$$\text{Inhibition Rate (\%)} = \left(1 - \frac{[B] - [N]}{[T] - [N]}\right) \times 100$$

[Notes]

1. "B" means the radioactivity of $^3$H-NECA bound in the presence of a test compound at a concentration shown in Table 2.
2. "T" means the radioactivity of $^3$H-NECA bound in the absence of test compounds.
3. "N" means the radioactivity of $^3$H-NECA bound in the presence of 100 μM of CPA.

The results are shown in Table 2. The Ki values shown in the table were calculated from the following equation:

$$Ki = \frac{IC_{50}}{1 + \frac{L}{Kd} + \frac{C}{Kc}}$$

[Notes]

$IC_{50}$: Concentration at an inhibition rate of 50%
L: Concentration of $^3$H-NECA
Kd: Dissociation constant of $^3$H-NECA
C: Concentration of CPA.
Kc: Inhibition constant of CPA

Table 2

| Compound No. | $A_1$ Receptor | | $A_2$ Receptor | | Ratio of Ki Values [ $A_2/A_1$ ] |
|---|---|---|---|---|---|
| | Inhibition (%)/ Concentration of Tested Compound [$10^{-5}/10^{-4}$M] | Ki (nM) | Inhibition (%)/ Concentration of Tested Compound [$10^{-5}/10^{-4}$M] | Ki (nM) | |
| 1 | 99/99 | 5.5 | 88/97 | 510 | 92.7 |
| 3 | 100/100 | 4.4 | 83/90 | 330 | 75.0 |
| 5 | 99/99 | 3.8 | 91/99 | 330 | 86.8 |
| 6 | 100/101 | 5.0 | 70/85 | 560 | 112 |
| 13 | 100/100 | 7.8 | 63/71 | 1,400 | 179 |
| 14 | 101/101 | 1.3 | 63/77 | 380 | 292 |
| 28 | 100/101 | 7.1 | 61/78 | 940 | 132 |
| 29 | 100/100 | 9.1 | 72/78 | 970 | 107 |
| XAC*1 | 98($10^{-6}$M) | 11 | 99/- | 21 | 1.91 |
| PD 115199*2 | 97/100 | 190 | 94/98 | 26 | 0.14 |
| CGS 15943*3 | 99/96 | 10 | 99/97 | 0.73 | 0.073 |
| Theophylline | 33/74 | 23,000 | 26/69 | 20,000 | 0.87 |

[Notes] *1: Xanthineaminecongener

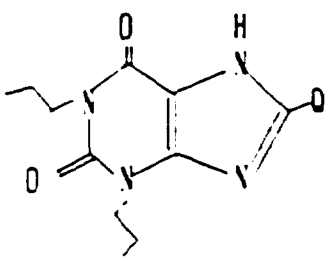

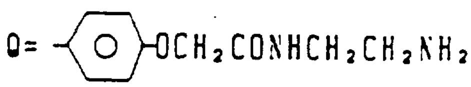

[Mol. Pharmacol., 29, 126 (1986)]

*2:

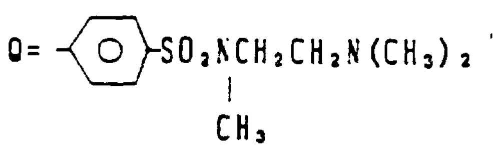

[Naunyn-Schmiedeberg's Arch. Pharmacol., 335, 64 (1987)]

*3:

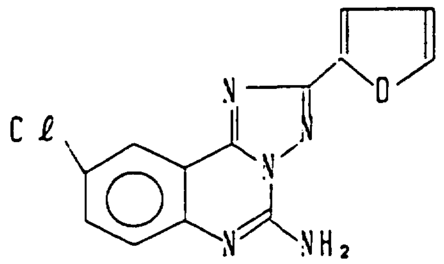

[J.Med. Chem., 31, 1014(1988)]

Test Example 3, diuretic effect

Wistar rats (male: 150 - 300 g) were starved for 18 hours prior to the administration of the test compounds. A test compound (25 mg/kg) and saline (25 mℓ/kg) were orally administered to test rats and only saline was administered to control rats. Three groups, each group consisting of 3 rats, were used for each test compound. Urine was collected for 6 hours after the administration. Urine volume was measured and the electrolytes ($Na^+$ and $K^+$) in the urine were determined with a flame photometer (775A, Hitachi Ltd., Japan). The results are shown in Table 3.

All parameters are expressed as relative values of control.

Table 3

| Compound No. | Increase in Urine (%) | Increase in $Na^+$excretion (%) | Increase in $K^+$ excretion (%) | $Na^+/K^+$ |
|---|---|---|---|---|
| (Control) | 0 | 0 | 0 | 1.00 |
| 1 | 106 | 73 | 36 | 1.27 |
| 2 | 87 | 109 | 67 | 1.25 |
| 3 | 154 | 137 | 29 | 1.84 |
| 4 | 113 | 106 | 27 | 1.63 |
| 5 | 88 | 109 | 32 | 1.58 |
| 6*3 | 330 | 252 | 87 | 1.88 |
| 8 | 82 | 138 | 22 | 1.95 |
| 12 | 108 | 103 | 32 | 1.54 |
| 13 | 129 | 186 | 37 | 2.09 |
| 14 | 315 | 244 | 68 | 2.05 |
| 16 | 141 | 191 | 38 | 2.12 |
| 17 | 155 | 107 | 51 | 1.37 |
| 24 | 112 | 125 | 61 | 1.40 |
| 29 | 123 | 137 | 65 | 1.43 |
| 30 | 112 | 126 | 50 | 1.50 |
| 31 | 115 | 126 | 56 | 1.44 |
| 32 | 100 | 114 | 41 | 1.51 |
| 33 | 99 | 105 | 40 | 1.47 |
| Aminophylline*1 (Reference compound) | 34 | 89 | 17 | 1.62 |
| Furosemide*2 (Reference compound) | 75 | 64 | 57 | 1.07 |

*1 The Merck Index, 10th edition, page 476 (1983)

*2 The Merck Index, 10th edition, page 4189 (1983)

*3 The amount of the administration: 6.25 mg.kg

Test Example 4, renal-protecting effect (glycerol-induced renal failure model).

A renal failure is a state wherein the renal function is lowered and the homeostasis of a body fluid can be no more maintained. It is known that an acute renal failure characteristic of uriniferous tubule disorder is caused by subcutaneous or intramuscular injection of glycerol to rats [Can. J. Physiol. Pharmacol., 65, 42 (1987)].

Male Wistar rats were kept deprived or water for 18 hours, and served for the test. A test compound was intraperitoneally administered to the rats (dosage: 1 mℓ/kg) and the rats were anesthetized with ether and 50% glycerol was subcutaneously administered (dosage: 0.8mℓ/100g) to the rats, pinching the dorsal skin. Twenty four hours after the administration of glycerol, the rats were anesthetized with ether and 5 mℓ of blood were collected from the abdominal aorta. The collected blood was allowed to stand for 30 minutes or longer and then centrifuged at 3,000 rpm for 10 minutes, and the amounts of the serum creatinine and urine-nitrogen (UN) contained in the serum were determined by auto analyzer (Olympus AU510) or measured by a creatinine test Wako (Jaffé method) and UN Test Wako (diacetylmonooxime direct method). Both are manufactured by Wako Pure Chemicals Co.

On the other hand, the left kidneys of the blood-sampled rats were removed and placed in formalin-filled vial bottles, and used as samples for the pathological examination.

According to the test results, Compound Nos. 1 - 5, 7, 8, 13, 14, 16, 17, 23, 25 and 31 significantly suppressed increases of the serum creatinine and of urine-nitrogen, when administered abdominally at a dosage of 0.01 - 10 mg/kg [i.p.] ($p < 0.05$) whereas XAC and aminophylline had a weak effect of suppressing the increase, and PD 115,199 and CGS15,943 were totally ineffective. On the contrary, furosemide showed a tendency to increase the serum creatinine. The pathological examination of removed kidneys indicates that compounds Nos. 1 - 5, 7, 8, 13, 14, 16, 17, 23, 25 and 31 also significantly improved the state of kidneys.

Test Example 5, effects on passive Schultz-Dale reaction

Male Hartley guinea pigs weighing 350 to 500 g were passively sensitized by intraperitoneal injection of rabbit anti-egg white albumin (EWA) serum prepared by the method of Koda, et al. [Folia pharmacol, Japon 66, 237 (1970)]. After 24 hours, the guinea pigs were stunned and exsanguinated, and then trachea were removed. The zig-zag strips of the trachea were prepared by the method of Emmerson, et al. [J. Pharm. Pharmacol., 31, 798 (1979)]. The strips were suspended in Krebs-Henseleit solution at 37°C airated with 95% $O_2$ and 5% $CO_2$, and incubated for one hour. Antigen (EWA) was then introduced in the solution (final concentration; 1μg/mℓ), and the contraction was measured by isotonictransducer (TD-112s, Nihon Kohden, Japan) and recorded on a recorder (Type 3066, Yokogawa-Hokushin Denki, Japan). After the contraction reached stable plateau, the compounds were cumulatively added in order to get concentration-relaxation curves.

Concentration of compounds to produce 50% relaxation ($IC_{50}$) was calculated from the regression curve, obtained from cumulative concentration-relaxation response. The results are shown in Table 4.

Table 4

| | Passive S-D Reaction MED (mg/kg) for Inhibiting | |
|---|---|---|
| Compound No. | $IC_{50}$ (μM) | Death Induced by PAF |
| 7 | 2.7 | >100 |
| 8 | 0.88 | 100 |
| 25 | 17.3 | - |
| 26 | 22.7 | - |
| Theophylline | 23 | 100 |

Test Example 6, effect of inhibiting death induced by Platelet-Activating Factor (PAF)

A test compound (100 mg/kg) was orally administered to dd strain mice (male animals, 28 to 32g) and 40 μg/kg of PAF (manufactured by Avanti Polar Lipids Co.) was administered via tail veins 1 hour after the administration according to the method of Carlson et al. [Agents and Actions, 21, 379 (1987)]. The mortality rates of compound-treated groups were compared with those of matched control groups, assessed during the same experimental session, by the Fisher's exact probability test. The cases wherein the level of significance (p value) is 0.05 or less are considered to be effective with respect to the inhibition. The above procedure was repeated, using the test compound in a decreasingly small quantity so as to find out the Minimum Effective Dosage (MED) wherein no significant difference be observed between the test and control groups.

The results are shown in Table 4.

Compound (I) or its physiologically acceptable salts can be used as such or in various medicament forms. The present pharmaceutical compositions can be prepared by uniformly mixing an effective amount of Compound (I) or its physiologically acceptable salts as an active component with a physiologically acceptable carrier. The pharmaceutical compositions are desirably in a unit dosage form applicable to oral or injection administration.

For the preparation of pharmaceutical compositions in an oral dosage form, some useful, physiologically acceptable carrier can be used. For example, liquid, orally administerable compositions such as suspension compositions or syrup compositions can be prepared with water, saccharides such as sucrose, sorbitol or fructose, glycols such as polyethyleneglycol or propylenglycol, oils such as sesame oil, olive oil or soybean oil an antiseptic such as p-hydroxybenzoic acid esters and/or flavors such as strawberry flavor or peppermint. Powder, pills, capsules and tablets can be prepared with vehicles such as lactose, glucose, sucrose or mannitol, disintegrators such as starch or sodium alginate, lubricants such as magnesium stearate or talc, binders such as polyvinyl alcohol, hydroxypropyl cellulose or gelatin, a surfactant such as fatty acid esters and/or a plasticizer such as glycerin.

Tablets and capsules are most useful units for oral administration because of easy administration. For the preparation of tablets or capsules, solid pharmaceutical carriers are used.

Injection solutions can be prepared with carriers such as distilled water, saline solutions glucose solutions, or a mixture of a saline solution and a glucose solution.

Effective dosage and number of administrations of Compound (I) or its physiologically acceptable salts depend on the administration route and ages, body weights or symptoms of patients, and it is preferred to usually administer Compound (I) at a dosage of 1 to 50 mg/kg per day in 3 to 4 portions.

Compound (I) and physiologically acceptable salts thereof can also be administered by inhalation in the form of aerosols, finely divided powders or sprayed mist. In the case of aerosol administration, the compounds according to the invention can be dissolved in an appropriate, physiologically acceptable solvent (e.g., ethyl alcohol) or a mixture of miscible solvents, and then admixed with a physiologically acceptable propellant. Such an aerosol composition can be charged in a pressure container equipped with an appropriate aerosol valve suited for the spraying of aerosol compositions. It can be preferable to use an aerosol valve which is capable of spraying a predetermined quantity of aerosol composition to provide an effective dosage thereof.

The present invention will be described below, by the following Examples and Reference Examples.

Example 1

8-[(1R*,4S*,5S*)-2-Bicyclo[2.2.1]hepten-5-yl]-1,3-dipropylxanthine (Compound 1), and 8-[(1R*,4S*,5R*)-2-bicyclo [2.2.1)hepten-5-yl)-1,3-dipropylxanthine (Compound 2)

At first, 2.57g (18.6 mmol) of bicyclo[2.2.1]-5-heptene-2-carboxylic acid and 3.06g (16.0 mmol) of 1-ethyl-3-(3-dimethylaminopropyl)carbodiimide hydrochloride were added to a solution of 3.00g (13.3 mmol) of 1,3-dipropyl-5,6-diaminouracil [US-A-2,607,295 and J. Org. Chem., 16, 1879 (1951)] in 60 mℓ of dioxane and 30 mℓ of water and the mixture was stirred at room temperature for 1 hour, while adjusting the pH to 5.5. The pH of the mixture was adjusted to 7.0, and the mixture was extracted with chloroform three times, and the extract was washed with a saturated aqueous sodium chloride solution and dried over anhydrous sodium sulfate. Then, the solvent was evaporated under reduced pressure and the residue was purified by silica gel column chromatography (eluent: 2.0% methanol/chloroform) to obtain 4.07g (yield: 88%) of amorphous 6-amino-5-(2-bicyclo[2,2,1]hepten-5-yl)carbonylamino-1,3-dipropyluracil.
NMR ($CDCl_3$, 90 MHz) δ(ppm): 7.20 (brs, 1H), 6.22-5.95 (m, 2H), 5.35 (brs, 2H), 4.00-3.65 (m, 4H), 3.52-2.80 (m, 3H) and 2.20-0.80 (m, 14H)

Then, 40 mℓ of dioxane and 40 mℓ of 2N sodium hydroxide aqueous solution were added to 3.99g (11.5 mmol) of the thus obtained compound and the mixture was refluxed under heating for 20 minutes. After cooling, the mixture was neutralized and extracted with chloroform three times. Then, the extract was washed with a saturated aqueous sodium chloride solution and dried over anhydrous sodium sulfate, and then the solvent was evaporated under reduced pressure. The residue was purified by silica gel column chromatography (eluent: 25% ethyl acetate/hexane) and recrystallized from cyclohexane to obtain 1.97g (yield: 52%) of the isolated Compound 1 as a white powder and 0.63g (yield: 18%) of the isolated Compound 2 as a white powder.

Compound 1:
Melting point: 121.6 - 122.8°C (recrystallized from isopropanol/water)
Rf value: 0.30 [TLC plate silica gel $60F_{254}$ (product of Merck Co., eluent: 30% ethylacetate/hexane]

| Elemental analysis: $C_{18}H_{24}N_4O_2$ | | | |
|---|---|---|---|
| Calculated (%): | C 65.83, | H 7.37, | N 17.06 |

(continued)

| Elemental analysis: $C_{18}H_{24}N_4O_2$ | | | |
|---|---|---|---|
| Found (%) : | C 65.71, | H 7.51, | N 16.78 |

IR (KBr) νmax ($cm^{-1}$): 1,698, 1,653, 1,497
NMR (DMSO-$d_6$) δ(ppm): 12.84 (s, 1H), 6.17 (dd, J= 3.2, 5.6 Hz, 1H), 5.72 (dd, J=2.7, 5.6 Hz, 1H), 3.91 (t, 2H), 3.82 (t, 2H), 3.43 (ddd, J=4.2, 4.2, 9.3 Hz, 1H), 3.28 (brs, 1H), 2.92 (brs, 1H), 2.08 (ddd, J=3.7, 9.3, 13.0 Hz, 1H), 1.75-1.50 (m, 5H), 1.45-1.35 (m, 2H) and 0.90-0.80 (m, 6H)

Compound 2:
Melting point: 167.6 - 168.0°C (recrystallized from ethanol/water)

| Elemental analysis: $C_{18}H_{24}N_4O_2$ | | | |
|---|---|---|---|
| Calculated (%): | C 65.83, | H 7.37, | N 17.06 |
| Found (%) : | C 66.03, | H 7.69, | N 17.09 |

Rf value: 0.46 (30% ethyl acetate/hexane)
IR (KBr) νmax ($cm^{-1}$): 1,695, 1,657, 1,495
NMR (DMSO-$d_6$) δ(ppm): 13.11 (brs, 1H), 6.21 (d, J=1.4 Hz, 2H), 3.95 (t, 2H), 3.84 (t, 2H), 2.96 (brs, 2H), 2.63 (ddd, J= 0.7, 4.2, 8.2 Hz, 1H), 2.10 (ddd, J= 4.2, 4.2, 11.5 Hz, 1H), 1.75 -1.45 (m, 5H), 1.35 - 1.22 (m, 2H), 0.92 - 0.80 (m, 6H).

Example 2

8-[(1R*,2S*,5S*)-Bicyclo[2,2,1]heptan-2-yl]-1,3-dipropylxanthine (Compound 3) and 8-[(1R*,2R*,5S*)-bicyclo[2.2.1) heptan-2-yl]-1,3-dipropylxanthine (Compound 4)

The substantially same operations as in Example 1 were repeated using 3.0g (13.3 mmol) of 1,3-dipropyl-5,6-diaminouracil and 2.61g (18.6 mmol) of bicyclo[2.2.1]heptane-2-carboxylic acid to obtain 4.31g (yield: 93%) of amorphous 6-amino-5-(bicyclo[2.2.1]heptan-2-yl)carbonylamino-1,3-dipropyluracil.
NMR ($CDCl_3$, 90 MHz) δ(ppm): 7.21 (brs, 1H), 5.40 (brs, 2H), 4.00 - 3.70 (m, 4H), 3.00 - 2.75 (m, 1H), and 2.65 - 0.75 (m, 20H)

The substantially same cyclization reaction as in Example 1 was performed using 4.30g (12.3 mmol) of the thus obtained compound, to obtain 3.05g (yield: 75%) of 8-bicyclo[2.2.1]heptan-2-yl)-1,3-dipropylxanthine [a mixture of (1R*, 2S*, 5S*) isomer (Compound 3) and (1R*, 2R*, 5S*) isomer (Compound 4)] as a white powder. The mixture was subjected to high performance liquid chromatography (HPLC) [column, R-354 (30 cm x 50 mm∅) (by Yamamura Kagaku K.K.); eluent, 85% methanol/water; flow rate, 50 mℓ/min.] to obtain 327 mg of the isolated Compound 3 and 442 mg of the isolated Compound 4.

Compound 3:
Melting point: 150.9 - 152.0°C (recrystallized from isopropanol/water)

| Elementary analysis: $C_{18}H_{26}N_4O_2$ | | | |
|---|---|---|---|
| Calculated (%): | C 65.43, | H 7.93, | N 16.96 |
| Found (%) : | C 65.41, | H 8.11, | N 17.00 |

IR (KBr) νmax ($cm^{-1}$): 1700, 1650, 1497.
NMR (DMSO-$d_6$) δ(ppm): 13.00 (brs, 1H), 3.97 (t, 2H), 3.84 (t, 2H), 3.21 (ddd, J=4.2, 4.2, 11.6 Hz, 1H), 2.55 (brs, 1H), 2.28 (brs, 1H), 1.90 - 1.22 (m, 11H), 1.15 -1.03 (m, 1H), 0.95 -0.82 (m, 6H).
HPLC [AM-312 (15 cm x 5 mm∅) (by Yamamura Kagaku K.K.), 70% acetonitrile-water, UV 254 nm, 1.0 mℓ/min]: Retention time; 12.7 min.

Compound 4:
Melting point: 139.7 - 142.9°C (recrystallized from isopropanol/water)

| Elementary analysis: $C_{18}H_{26}N_4O_2$ | | | |
|---|---|---|---|
| Calculated (%): | C 65.43, | H 7.93, | N 16.96 |
| Found (%) : | C 65.66, | H 8.29, | N 16.90 |

IR (KBr) νmax ($cm^{-1}$): 1702, 1650, 1494.
NMR (DMSO-$d_6$) δ(ppm): 12.99 (brs, 1H), 3.94 (t, 2H), 3.83 (t, 2H), 2.79 (dd, J=4.9,8.5 Hz, 1H), 2.39 (brs, 1H), 2.31 (brs, 1H), 2.08 - 1.96 (m, 1H), 1.80 - 1.45 (m, 8H), 1.38 - 1.12 (m, 3H), 0.95 - 0.80 (m, 6H).
HPLC [AM-312 (15 cm x 5 mm∅) (by Yamamura Kagaku K.K.) 70% acetonitrile-water, UV 254 nm, 1.0 mℓ/min]: Retention time; 13.9 min.

In Examples 3 to 9 described below, the desired compounds were obtained in the substantially same operations as in Example 1, except that a corresponding carboxylic acid was used instead of bicyclo[2.2.1]-5-heptene-2-carboxylic acid. In those examples, intermediates obtained were used in the subsequent cyclization reactions without being isolated or purified.

Example 3

8-[(1R*,2R*,5R*)-Bicyclo[3.3.0]octan-2-yl]-1,3-dipropylxanthine (Compound 5) and 8-[(1R*,2S*,5R*)-bicyclo[3.3.0]octan-2-yl]-1,3-dipropylxanthine (Compound 6)

The substantially same operations as in Example 1 were repeated using 4.55 mℓ (31.9 mmol) of bicyclo[3.3.0]octane-2-carboxylic acid, and the following two compounds were obtained.

Compound 5:
Yield: 4.30g (Yield, 47%; white plate crystal)
Melting point: 100.1 - 101.6°C (recrystallized from heptane)
Rf value: 0.53 (30% ethyl acetate/hexane)

| Elementary analysis: $C_{19}H_{28}N_4O_2$ | | | |
|---|---|---|---|
| Calculated (%): | C 66.25, | H 8.19, | N 16.27 |
| Found (%) : | C 66.07, | H 8.43, | N 16.61 |

IR (KBr) νmax ($cm^{-1}$): 1,699, 1,653 and 1,499
NMR(DMSO-$d_6$) δ(ppm): 13.12 (brs, 1H), 3.94 (t, 2H), 3.83 (t, 2H), 2.75 - 2.50 (m, 3H), 2.10 - 1.45 (m, 12H), 1.42 -1.35 (m, 1H), 1.30 - 1.15 (m, 1H), 0.95 - 0.85 (m, 6H)
$^{13}$C-NMR (CDCℓ$_3$) δ(ppm): 159.1, 155.7, 151.1, 149.4, 106.7, 50.4, 47.6, 45.3, 43.4, 43.2, 34.4, 34.1, 33.6, 32.1, 25.1, 21.4, 11.4, 11.2.

Compound 6:
Yield: 359 mg (Yield, 3.9%; white plate crystal)
Melting point: 118.4 - 120.0°C (recrystallized from heptane)
Rf value: 0.40 (30% ethyl acetate/hexane)

| Elementary analysis: $C_{19}H_{28}N_4O_2$ | | | |
|---|---|---|---|
| Calculated (%): | C 66.25, | H 8.19, | N 16.27 |
| Found (%) : | C 66.20, | H 8.63, | N 16.31 |

IR (KBr) νmax ($cm^{-1}$): 1,699, 1,652 and 1,497
NMR (CDCℓ$_3$) δ(ppm): 12.30 (brs, 1H), 4.11 (t, 2H), 4.02 (t, 2H), 3.30 (ddd, 1H, J=6.8, 14 Hz), 3.00 - 2.85 (m, 1H), 2.70 - 2.53 (m, 1H), 2.25 - 0.90 (m, 20H)
$^{13}$C-NMR (CDCℓ$_3$) δ(ppm): 157.0, 155.5, 151.2, 149.2, 106.5, 47.6, 45.2, 44.0, 43.3, 42.9, 35.4, 32.5, 29.7, 27.5, 27.4, 21.4, 21.4, 11.4, 11.2.
MS (m/e) relative intensity: 344 ($M^+$, 100), 302 (28), 260 (18), 250 (23) and 230 (18)

Example 4

8-[1-methyl-2-(4-pyridyl)ethyl]-1,3-dipropylxanthine (Compound 7)

Overall yield: 79% (White needle crystal)
Melting point: 214.9 - 217.3°C

| Elementary analysis: $C_{19}H_{25}N_5O_2 \cdot HCl \cdot 0.1H_2O$ | | | |
|---|---|---|---|
| Calculated (%): | C 57.74, | H 6.64, | N 17.72 |
| Found (%) : | C 57.79, | H 6.54, | N 17.63 |

IR (KBr) νmax ($cm^{-1}$): 1,704, 1,669 and 1,637
NMR (DMSO-$d_6$) δ(ppm): 13.50 - 12.80 (brs, 1H), 8.79 (d, 2H, J=6.1 Hz), 7.84 (d, 2H, J=6.1 Hz), 3.90 (t, 2H), 3.81 (t, 2H), 3.50 - 3.20 (m, 3H), 1.70 - 1.50 (m, 4H), 1.33 (d, 3H, J=6.7 Hz), 0.85 (t, 3H), 0.82 (t, 3H)

Example 5

8-[1-Methyl-2-(2-methylthiazol-4-yl)ethyl]-1,3-dipropylxanthine (Compound 8)

Overall yield: 70% (White plate crystal)
Melting point: 137.6 - 139.2°C (recrystallized from cyclohexane)

| Elementary analysis: $C_{18}H_{25}N_5O_2S$ | | | |
|---|---|---|---|
| Calculated (%): | C 57.58, | H 6.71, | N 18.65 |
| Found (%) : | C 57.75, | H 6.72, | N 18.48 |

IR (KBr) νmax ($cm^{-1}$): 1,698, 1,659 and 1,499
NMR (DMSO-$d_6$) δ(ppm): 13.11 (brs, 1H), 7.00 (s, 1H), 3.94 (t, 2H), 3.83 (t, 2H), 3.45 - 3.10 (m, 3H), 2.92 (dd, 1H, J=6.8, 14.2 Hz), 2.59 (s, 3H), 1.75 - 1.50 (m, 4H), 1.23 (d, 3H, J=6.8 Hz), 0.95 - 0.80 (m, 6H)

Example 6

8-(Benzo[b]thiophen-2-yl)-1,3-dipropylxanthine (Compound 9)

Overall yield: 61% (White needle crystal)
Melting point: 307.9 - 309.1°C (recrystallized from ethanol)

| Elementary analysis: $C_{19}H_{20}N_4O_2S$ | | | |
|---|---|---|---|
| Calculated (%): | C 61.94, | H 5.47, | N 15.21 |
| Found (%) : | C 61.91, | H 5.44, | N 15.15 |

IR (KBr) νmax ($cm^{-1}$): 1,699, 1,642 and 1,537
NMR (DMSO-$d_6$) δ (ppm): 8.19 (s, 1H), 8.05 - 7.85 (m, 2H), 7.50 - 7.40 (m, 2H), 4.00 (t, 2H), 3.87 (t, 2H), 1.85 - 1.50 (m, 4H), 1.00 - 0.80 (m, 6H)

Example 7

8-(Benzo[b]furan-2-yl)-1,3-dipropylxanthine (Compound 10)

Overall yield: 71% (White needle crystal)
Melting point: 282.1 - 283.9°C (recrystallized from ethanol)

| Elementary analysis: $C_{19}H_{20}N_4O_3$ | | | |
|---|---|---|---|
| Calculated (%): | C 64.76, | H 5.72, | N 15.90 |

(continued)

| Elementary analysis: $C_{19}H_{20}N_4O_3$ | | | |
|---|---|---|---|
| Found (%) : | C 64.80, | H 5.72, | N 15.77 |

IR (KBr) νmax ($cm^{-1}$): 1,700 and 1,648
NMR (DMSO-$d_6$) δ(ppm): 14.30 (brs, 1H), 7.80 - 7.65 (m, 2H), 7.68 (s, 1H), 7.50 - 7.30 (m, 2H), 4.02 (t, 2H), 3.88 (t, 2H), 1.85 - 1.50 (m, 4H), 1.00 - 0.80 (m, 6H)

Example 8

8-(3-Methylinden-2-yl)-1,3-dipropylxanthine (Compound 11)

Overall yield: 36% (Light yellow plate crystal)
Melting point: 268.1 - 269.9°C (recrystallized from ethanol)

| Elementary analysis: $C_{21}H_{24}N_4O_2$ | | | |
|---|---|---|---|
| Calculated (%): | C 69.21, | H 6.64, | N 15.37 |
| Found (%) : | C 69.40, | H 6.72, | N 15.34 |

IR (KBr) νmax ($cm^{-1}$): 1,690, 1,641 and 1,485
NMR (DMSO-$d_6$) δ(ppm): 13.33 (brs, 1H), 7.55 - 7.45 (m, 2H), 7.40 - 7.25 (m, 2H), 4.04 (t, 2H), 3.88 (s, 2H), 3.90 - 3.80 (m, 2H), 2.61 (s, 3H), 1.85 - 1.50 (m, 4H), 0.95 - 0.80 (m, 6H)

Example 9

8-(2-Aminothiazol-4-yl)methyl-1,3-dipropylxanthine (Compound 12)

Overall yield: 94% (Light yellow plate crystal)
Melting point: 282.5 - 284.3°C (recrystallized from isopropanol)
IR (KBr) νmax ($cm^{-1}$): 1,697, 1,660, 1,523 and 1,500
NMR (DMSO-$d_6$) δ (ppm): 13.28 (brs, 1H), 6.89 (brs, 2H), 6.23 (s, 1H), 4.00 - 3.80 (m, 4H), 3.86 (s, 2H), 1.80 -1.50 (m, 4H), 0.95 - 0.80 (m, 6H)
MS (m/e) (Relative intensity): 348 ($M^+$, 100), 306 (51), 277 (26), 264 (47), 248 (28), 234 (86) and 113 (38)

Example 10

8-(Noradamantan-3-yl)-1,3-dipropylxanthine (Compound 14)

At first, 1.62g (9.74 mmol) of 3-noradamantanecarboxylic acid was dissolved in 30 mℓ of pyridine, and 0.78 mℓ (10.7 mmol) of thionyl chloride was gradually added thereto at 0°C. After the mixture was stirred for 30 minutes at room temperature, 2.00 g (8.85 mmol) of 1,3-dipropyl-5,6-diaminouracil were gradually added thereto at 0°C. After stirring for 30 minutes at 0°C, the mixture was treated in the same procedure as in Example 29, and the residue was subjected to silica gel column chromatography (eluent: 1% methanol/chloroform) to obtain 3.55g (yield: 100%) of amorphous 6-amino-5-(noradamantane-3-carbonylamino)-1,3-dipropyluracil.
NMR (90 MHz; $CDCℓ_3$) δ(ppm): 7.38 (brs, 1H), 5.62 (brs, 2H), 4.00 - 3.70 (m, 4H), 2.90 - 2.60 (m, 1H), 2.40 - 1.30 (m, 16H), 1.10 - 0.80 (m, 6H)

The substantially same cyclization reaction as in Example 29 was performed by reacting 2.90g (7.55 mmol) of the thus obtained compound with phosphorus oxychloride to obtain 509 mg (yield: 14%) of the isolated Compound 14 as white needle crystals.
Melting point: 190.0 - 191.0°C (recrystallized from heptane)

| Elementary analysis: $C_{20}H_{28}N_4O_2$ | | | |
|---|---|---|---|
| Calculated (%): | C 67.39, | H 7.92, | N 15.72 |
| Found (%) : | C 67.41, | H 7.62, | N 15.78 |

IR (KBr) νmax ($cm^{-1}$): 1,699, 1,651 and 1,499
NMR (DMSO-$d_6$) δ(ppm): 12.97 (s, 1H), 3.95(t, 2H), 3.85 (t, 2H), 2.70 - 2.60 (m, 1H), 2.35 - 2.26 (m, 2H), 2.20 - 2.10 (m, 2H), 1.95 - 1.82 (m, 4H), 1.80 -1.50 (m, 8H), 0.95 - 0.80 (m, 6H)
$^{13}$C-NMR (DMSO-$d_6$) δ (ppm): 159.9, 153.9, 150.7, 147.6, 106.6, 48.8, 48.2, 45.1, 44.2 , 43.2, 41.9, 36.9, 34.1, 20.8, 11.1, 10.9.

Example 11

8-(Adamantan-1-yl)methyl-1,3-dipropylxanthine (Compound 15)

At first 2.00g (8.85 mmol) of 1,3-dipropyl-5,6-diaminouracil and 2.06 g (10.6 mmol) of 1-adamantaneacetic acid were condensed according to the procedure of Example 1 using 1-ethyl-3-(3-dimethylaminopropyl)carbodiimide to obtain 4.02 g (yield: 100%) of amorphous 6-amino-5-(adamantan-1-yl)acetylamino-1,3-dipropyluracil as a crude product.
NMR ($CDCℓ_3$) δ (ppm): 7.42 (brs, 1H), 5.47 (brs, 2H), 4.00 - 3.70 (m, 4H), 2.20 - 1.20 (m, 21H), 1.10 - 0.80 (m, 6H)

The substantially same cyclization reaction as in Example 29 was performed by reacting 3.95g of the thus obtained compound with phosphorus oxychloride to obtain 1.66g (overall yield: 49%) of the isolated Compound 15 as white needle crystals.
Melting point: 177.7 - 179.5°C (recrystallized from isopropanol/water)

| Elementary analysis: $C_{22}H_{32}N_4O_2$ | | | |
|---|---|---|---|
| Calculated (%): | C 68.72, | H 8.39, | N 14.57 |
| Found (%) : | C 68.71, | H 8.74, | N 14.70 |

IR (KBr) νmax ($cm^{-1}$): 1,704, 1,648 and 1,498
NMR (DMSO-$d_6$) δ(ppm): 13.06 (brs, 1H), 3.95 (t, 2H), 3.83 (t, 2H), 3.40 - 3.25 (m, 2H), 2.43 (brs, 2H), 1.90 (brs, 3H), 1.80 - 1.45 (m, 16H), 0.95 - 0.85 (m, 6H)

Example 12

8-[(1R*,2R*,5S*)-Bicyclo[2.2.1]heptan-2-yl]methyl-1,3-dipropylxanthine (Compound 16)

Compound 16 was obtained in the same procedure as in Example 11, except for using 1.54 mℓ (10.6 mmol) of (1R*,2R*,5S*)-bicyclo[2.2.1]heptane-2-acetic acid in place of 1-adamantaneacetic acid.
Yield: 1.22 g (White needle crystal; overall yield, 40%)
Melting point: 119.9 - 121.4°C (recrystallized from isopropanol/water)

| Elementary analysis: $C_{19}H_{28}N_4O_2$ | | | |
|---|---|---|---|
| Calculated (%): | C 66.25, | H 8.19, | N 16.27 |
| Found (%) : | C 66.29, | H 8.32, | N 16.06 |

IR (KBr) νmax ($cm^{-1}$): 1,699, 1,654 and 1,502
NMR ($CDCℓ_3$) δ(ppm): 12.83 (brs, 1H), 4.15 - 4.00 (m, 4H), 2.81 (dd, 1H, J=7.8, 14.2 Hz), 2.66 (dd, 1H, J=7.8, 14.2 Hz), 2.26 (brs, 1H), 2.15 - 2.00 (m, 2H), 1.95 -1.65 (m, 4H), 1.60 - 1.40 (m, 4H), 1.30 - 0.90 (m, 10H)

Example 13

8-[(1R*,4S*, 5S*)-2-Bicyclo[2.2.1]hepten-5-yl]-1,3-dipropyl-7-methylxanthine (Compound 17)

At first 1.00 g (3.05 mmol) of 8-[(1R*,4S*,5S*)-2-bicyclo[2.2.1]hepten-5-yl]-1,3-dipropylxanthine (Compound 1) prepared in Example 1 was dissolved in 30 mℓ of N,N′-dimethylformamide, and 1.05 g (7.61 mmol) of potassium carbonate and 0.38 mℓ (6.10 mmol) of methyl iodide were added thereto. After the mixture was stirring at 50°C for 30 minutes under argon atmosphere, insoluble materials were filtered off, and the filtrate was concentrated under reduced pressure. The residue was poured into 200 mℓ of water and the mixture was extracted with chloroform three times. The organic layer was combined, and the extract was washed with water and then with saturated aqueous sodium chloride, and dried over anhydrous sodium sulfate and then the solvent was evaporated under reduced pressure. The

residue was subjected to silica gel column chromatography (eluent: 20% ethyl acetate/hexane) to obtain 1.05g (yield: 100%) of the isolated Compound 17 as a light yellow powder.
Melting point: 99.8 - 103.1°C (recrystallized from acetone/water)

| Elementary analysis: $C_{19}H_{26}N_4O_2$ | | | |
|---|---|---|---|
| Calculated (%): | C 66.64, | H 7.65, | N 16.36 |
| Found (%) : | C 66.60, | H 7.97, | N 16.55 |

IR (KBr) νmax ($cm^{-1}$): 1698, 1666, 1652, 1445.
NMR ($CDC\ell_3$) δ(ppm): 6.19 (dd, J=3.0, 5.6 Hz, 1H), 5.87 (dd, J=2.8, 5.6 Hz, 1H), 4.01 (t, 2H), 3.95 (t, 2H), 3.94 (s, 3H), 3.36 - 3.28 (m, 2H), 3.00 (brs, 1H), 2.19 (ddd, J=3.9, 9.3, 11.5 Hz, 1H), 1.84 - 1.50 (m, 6H), 1.45 -1.40 (m, 1H), 1.00 -0.90 (m, 6H).

Example 14

8-[(1R*,2R*,5R*)-Bicyclo[3.3.0]octan-2-yl]-1,3-dipropyl-7-methylxanthine (Compound 18)

The substantially same operations as in Example 13 were repeated except for using 1.00 g (2.90 mmol) of 8-[(1R*,2R*,5R*)-bicyclo[3.3.0]octan-2-yl]-1,3-dipropylxanthine (Compound 5) prepared in Example 3 to obtain 1.05 g (yield: 100%) of the isolated Compound 18 as a white powder.
Melting point: 94.7 - 97.0°C (recrystallized from ethanol/water)

| Elementary analysis: $C_{20}H_{30}N_4O_2$ | | | |
|---|---|---|---|
| Calculated (%): | C 67.01, | H 8.44, | N 15.63 |
| Found (%) : | C 66.93, | H 8.20, | N 15.68 |

IR (KBr) νmax ($cm^{-1}$): 1702, 1653.
NMR ($CDC\ell_3$) δ(ppm): 4.05 (t, 2H), 3.96 (t, 2H), 3.93 (s, 3H), 2.96 - 2.84 (m, 1H), 2.80 - 2.64 (m, 2H), 2.20 - 1.60 (m, 11H), 1.48 - 1.25 (m, 3H), 1.02 - 0.94 (m, 6H).

Example 15

1,3-Dipropyl-7-methyl-8-(noradamantan-3-yl)xanthine (Compound 20)

The substantially same operations as in Example 13 were repeated except for using 2.20 g (6.18 mmol) of 8-(noradamantan-3-yl)-1,3-dipropylxanthine (Compound 14) prepared in Example 10 to obtain 1.06g (yield: 46%) of the isolated Compound 20 as white needle crystals.
Melting point: 123.2 - 124.8°C (recrystallized from ethanol/water)

| Elementary analysis: $C_{21}H_{30}N_4O_2$ | | | |
|---|---|---|---|
| Calculated (%): | C 68.08, | H 8.16, | N 15.12 |
| Found (%) : | C 67.93, | H 8.23, | N 15.44 |

IR (KBr) νmax ($cm^{-1}$): 1698, 1661.
NMR ($CDC\ell_3$) δ(ppm): 4.05 (t, 2H), 4.01 (s, 3H), 3.96 (t, 2H), 2.98(t, 2H), 2.40 (brs, 2H), 2.25 - 2.17 (m,2H), 2.11 - 1.90 (m, 6H).

Example 16

7-Ethyl-1,3-dipropyl-8-(noradamantan-3-yl)xanthine (Compound 21)

The substantially same operations as in Example 13 were repeated except for using 1.40 g (3.93 mmol) of 8-(noradamantan-3-yl)-1,3-dipropylxanthine (Compound 14) prepared in Example 10 and 0.62 mℓ (7.87 mmol) of ethyl iodide to obtain 410 mg (yield: 27%) of the isolated Compound 21 as white crystals.
Melting Point: 91.3 - 92.4°C (recrystallized from acetonitrile)

| Elementary analysis: $C_{22}H_{32}N_4O_2$ | | | |
|---|---|---|---|
| Calculated (%): | C 68.71, | H 8.38, | N 14.57 |
| Found (%) : | C 68.88, | H 8.59, | N 14.69 |

IR (KBr) νmax ($cm^{-1}$): 1698, 1661, 1535.
NMR (CDCℓ$_3$, 90 MHz) δ(ppm): 4.34 (g, J= 7.0 Hz, 2H), 4.20 -3.86 (m, 4H), 3.03 (t, 1H), 2.50 - 1.40 (m, 16H), 1.50 (t, J=7.0 Hz, 3H), 1.15 - 0.85 (m, 6H).

Example 17

8-(Noradamantan-3-yl)-1,3,7-tripropylxanthine (Compound 22)

The substantially same operations as in Example 13 were repeated except for using 1.50 g (4.21 mmol) of 8-(noradamantan-3-yl)-1,3-dipropylxanthine (Compound 14) prepared in Example 10 and 0.82 mℓ (8.43 mmol) of propyl iodide to obtain 1.40g (yield: 84%) of the isolated Compound 22 as white crystals.
Melting point: 111.2 - 112.2°C (Recrystallized from ethanol/water)

| Elementary analysis: $C_{23}H_{34}N_4O_2$ | | | |
|---|---|---|---|
| Calculated (%): | C 69.31, | H 8.59, | N 14.05 |
| Found (%) : | C 69.29, | H 8.69, | N 14.57 |

IR (KBr) νmax ($cm^{-1}$): 1700, 1662, 1536.
NMR (CDCℓ$_3$, 90 MHz) δ(ppm): 4.30 - 3.85 (m, 6H), 3.05 (t, 1H), 2.50 - 1.50 (m, 18H), 1.20 - 0.85 (m, 6H).

Example 18

8-[(1R*,2R*,5R*)-Bicyclo[3.3.0]octan-2-yl]-3-propylxanthine (Compound 23)

At first, 5.00 g (27.2 mmol) of 5,6-diaminouracil and
3.88 mℓ (27.2 mmol) of bicyclo[3.3.0]octan-2-carboxylic acid,
8.40g (40.8 mmol) of N,N′-dicyclohexylcarbodiimide were suspended in JP-A-57,517/80 100 mℓ of N,N′-dimethylformamide and then
5.00 g (32.6 mmol) of N-hydroxybenzotriazole were added thereto. After the mixture was stirred overnight at room temperature, insoluble materials were filtered off, and the filtrate was concentrated under reduced pressure. 100 mℓ of aqueous 4N sodium hydroxide solution were added to the residue, and the mixture was refluxed under heating for 20 minutes. After cooling, the mixture was neutralized with concentrated hydrochloric acid, and the precipitated crystals were collected by filtration. 500 mℓ of water were added to the resulting crystals, and the mixture was extracted with chloroform three times. The combined extract was washed with saturated aqueous sodium chloride and dried over anhydrous sodium sulfate, and then the solvent was evaporated under reduced pressure. The obtained crude crystals were recrystallized from ethanol-water to obtain 3.68g (yield: 45%) of the isolated Compound 23 as white needle crystals. Melting point: 252.8 - 257.9°C (recrystallized from ethanol/water)

| Elementary analysis: $C_{16}H_{22}N_4O_2$ | | | |
|---|---|---|---|
| Calculated (%): | C 63.55, | H 7.33, | N 18.53 |
| Found (%) : | C 63.40, | H 7.67, | N 18.88 |

IR (KBr) νmax ($cm^{-1}$): 1700, 1678.
NMR (DMSO-$d_6$) δ(ppm): 13.03 (brs, 1H), 10.88 (brs, 1H), 3.87 (t, 2H), 2.72 - 2.50 (m, 3H), 2.10 - 1.15 (m, 12H), 0.87 (t, 3H).

Example 19

8-[(1R*,2R*,5R*)-Bicyclo[3.3.0]octan-2-yl]-1,3-dipropyl-2-thioxanthine (Compound 24)

The substantially same operations as in Example 1 were repeated except for using 2.00 g (8.26 mmol) of 5,6-diamino-1,3-dipropyl-2-thiouracil [J. Med. Chem., 32, 1873 (1989)] and 1.42 mℓ (9.92 mmol) of bicyclo[3.3.0]octan-2-carboxylic acid to obtain 1.70 g (overall yield: 57%) of the isolated Compound 24 as white crystals.
Melting point: 135.1 - 137.2°C (recrystallized from ethanol)

| Elementary analysis: $C_{19}H_{28}N_4OS$ | | | |
|---|---|---|---|
| Calculated (%): | C 63.30, | H 7.83, | N 15.54 |
| Found (%) : | C 63.54, | H 8.14, | N 15.59 |

IR (KBr) νmax ($cm^{-1}$): 1688, 1493.
NMR ($CDCℓ_3$) δ(ppm): 12.68 (brs, 1H), 4.68 (t, 2H), 4.63 (t, 2H), 2.92 - 2.65 (m, 3H), 2.25 - 1.53 (m, 12H), 1.50 - 1.43 (m, 1H), 1.41 - 1.22 (m, 1H), 1.15 - 0.98 (m, 6H).
HPLC [AM-312 (15 cm x 5 mmØ) (by Yamamura Kagaku K.K.), 70% acetonitrile-water, UV 254 nm, 2.0 mℓ/min]: Retention time; 14.4 min.

Example 20

8-(Noradamantan-3-yl)-3-propylxanthine (Compound 25)

The substantially same operations as in Example 10 were repeated except for using 2.00 g (10.9 mmol) of 1-propyl-5,6-diaminouracil to obtain 2.27 g (yield: 63%) of 6-amino-5-(noradamantane-3-carbonylamino)-3-propyluracil as a light yellow powder.
NMR ($DMSO-d_6$, 90 MHz) δ (ppm): 10.47 (brs. 1H), 7.63 (brs, 1H), 6.23 (brs, 2H), 3.78 (t, 2H), 2.71 (t, 1H), 2.32 - 1.38 (m, 14H), 0.89 (t, 3H).

The substantially same cyclization reaction as in Example 1 was performed using 2.16 g (6.51 mmol) of the thus obtained compound and 40 mℓ of a 2N sodium hydroxide aqueous solution to obtain 1.85 g (yield: 91%) of the isolated Compound 25 as white crystals.
Melting point: >290°C (from dioxane/water)

| Elementary analysis: $C_{17}H_{22}N_4O_2$ | | | |
|---|---|---|---|
| Calculated (%): | C 64.94, | H 7.05, | N 17.82 |
| Found (%) : | C 64.65, | H 7.20, | N 18.00 |

IR (KBr) νmax ($cm^{-1}$): 1698, 1660, 1500.
NMR ($DMSO-d_6$, 90 MHz) δ(ppm): 12.42 (brs, 1H), 11.90 (brs, 1H), 4.08 (t, 2H), 2.77 (t, 1H) 2.55 - 1.40 (m, 14H), 1.00 (t, 3H).

Example 21

8-(Noradamantan-3-yl)-3-propyl-6-thioxanthine (Compound 26)

At first, 20.0 g (63.7 mmol) of 8-(noradamantan-3-yl)-3-propylxanthine (Compound 25) prepared in Example 20 were dissolved in 370 mℓ of pyridine, and 23.1 g (104 mmol) of phosphorus pentasulfide were added thereto. The mixture was refluxed under heating for 4 hours and poured into ice water, and the solid substances deposited were collected by filtration. The filtrate was concentrated under reduced pressure, and the solid substances deposited were again collected by filtration. The deposited solid substances were combined and dissolved in 200 mℓ of 2N sodium hydroxide solution, and insoluble materials were filtered off. The filtrate was neutralized with concentrated hydrochloric acid, and the deposited crystals were collected by filtration. The crude crystals were recrystallized from ethanol-water to obtain 11.7g (yield: 56%) of the isolated Compound 26 as light yellow needle crystals.
Melting point: 214.2 - 216.0°C (recrystallized from ethanol/water)

| Elementary analysis: $C_{17}H_{22}N_4OS\cdot 1/5H_2O$ | | | |
|---|---|---|---|
| Calculated (%): | C 61.12, | H 6.76, | N 16.77 |
| Found (%) : | C 61.12, | H 6.82, | N 16.95 |

IR (KBr) νmax ($cm^{-1}$): 1668, 1595.
NMR ($CDC\ell_3$, 90 MHz) δ(ppm): 10.14 (brs, 1H), 9.43 (brs, 1H), 4.05 (t, 2H), 2.73 (t, 1H), 2.68 - 1.40 (m, 14H), 0.98 (t, 3H).

Example 22

8-(Noradamantan-3-yl)-1,3-dimethylxanthine (Compound 27)

The substantially same operations as in Example 10 were repeated except for using 3.00g (17.6 mmol) of 1,3-dimethyl-5,6-diaminouracil instead of 1,3-dipropyl-5,6-diaminouracil [J. Am. Chem. Soc., 76, 2798 (1954)] to obtain 3.61g (yield: 65%) of 6-amino-5-(noradamantane-3-carbonylamino)-1,3-dimethyluracil as a light yellow powder.
NMR (DMSO-$d_6$, 90 MHz) δ (ppm): 7.68 (brs, 1H), 6.28 (brs, 2H), 3.30 (s, 3H), 3.11 (s, 3H), 2.71 (t, 1H), 2.66 - 1.40 (m, 12H).

The substantially same cyclization reaction as in Example 1 was performed except for using 3.60g (11.3 mmol) of the thus obtained compound to obtain 2.41g (yield: 71%) of the isolated Compound 27 as white crystals.
Melting point: >295°C (recrystallized from ethanol/water)

Elementary analysis: $C_{16}H_{20}N_4O_2$
Calculated (%): C 63.98, H 6.71, N 18.65
Found (%) : C 63.97, H 6.78, N 18.89

IR (KBr) νmax ($cm^{-1}$): 1719, 1656, 1649, 1503.
NMR ($CDC\ell_3$, 90 MHz) δ (ppm): 11.93 (brs, 1H), 3.62 (s, 3H), 3.46 (s, 3H), 2.79 (t, 1H), 2.52 - 1.60 (m, 12H).

Example 23

8-(Noradamantan-3-yl)-1,3-diethylxanthine (Compound 28)

The substantially same operations as in Example 10 were repeated using 2.0 g (10.1 mmol) of 1,3-diethyl-5,6-diaminouracil [J. Am. Chem. Soc., 75, 114 (1953)] to obtain 2.01 g (yield: 58%) of 6-amino-5-(noradamantane-3-carbonylamino)-1,3-diethyluracil as a light yellow powder.
NMR ($CDC\ell_3$, 90 MHz) δ (ppm): 7.35 (brs, 1H), 5.61 (brs, 2H), 4.18 - 3.85 (m, 4H), 2.76 (t, 1H), 2.50 - 1.10 (m, 18H).

The substantially same cyclization reaction as in Example 1 was performed except for using 1.90g (5.49 mmol) of the thus obtained compound to obtain 1.58g (yield: 88%) of the isolated Compound 28 as white crystals.
Melting point: 259.8 - 263.1°C (recrystallized from ethanol/water)

| Elementary analysis: $C_{18}H_{24}N_4O_2$ | | | |
|---|---|---|---|
| Calculated (%): | C 65.83, | H 7.36, | N 17.05 |
| Found (%) : | C 65.99, | H 7.51, | N 17.30 |

IR (KBr) νmax ($cm^{-1}$): 1704, 1646, 1497.
NMR ($CDC\ell_3$, 90 MHz) δ (ppm): 11.93 (brs, 1H), 4.40 - 3.98 (m, 4H), 2.83 (t, 1H), 2.60 - 1.60 (m, 16H), 1.50 -1.18 (m, 6H).

Example 24

8-(Noradamantan-3-yl)-1,3-dibutylxanthine (Compound 29)

The substantially same operations as in Example 10 were repeated except for using 1.70g (6.69 mmol) of 1,3-dibutyl-5,6-diaminouracil (US-A-2,607,295) to obtain 2.42g (yield: 90%) of amorphous 6-amino-5-(noradamantane-3-carbonylamino)-1,3-dibutyluracil.
NMR ($CDC\ell_3$, 90 MHz) δ (ppm): 7.40 (brs, 1H), 5.59 (brs, 2H), 4.05 - 3.76 (m, 4H), 2.76 (t, 1H), 2.50 - 0.80 (m, 24H).

The substantially same cyclization reaction as in Example 1 was performed using 2.08g (5.17 mmol) of the thus obtained compound to obtain 1.87g (yield: 94%) of the isolated Compound 29 as a light yellow powder.
Melting point: 159.7 - 161.0°C (recrystallized from ethanol/water)

| Elementary analysis: $C_{22}H_{32}N_4O_2$ | | | |
|---|---|---|---|
| Calculated (%): | C 68.71, | H 8.38, | N 14.57 |
| Found (%) : | C 68.69, | H 8.23, | N 14.81 |

IR (KBr) νmax ($cm^{-1}$): 1704, 1651, 1498.
NMR ($CDC\ell_3$, 90 MHz) δ (ppm) : 11.67 (brs, 1H), 4.28 - 3.90 (m, 4H), 2.82 (t, 1H), 2.62 - 1.19 (m, 18H), 1.15 - 0.80 (m, 6H).

Example 25

8-(Noradamantan-3-yl)-3-isobutyl-1-methylxanthine (Compound 30)

The substantially same operations as in Example 10 were repeated except for using 1.87g (8.81 mmol) of 1-isobutyl-3-methyl-5,6-diaminouracil [Methods in Enzymology, 159, 489 (1988)] to obtain 2.63g (yield: 83%) of 6-amino-5-(noradamantane-3-carbonylamino)-1-isobutyl-3-methyluracil as a white powder.
NMR ($CDC\ell_3$, 90 MHz) δ (ppm): 7.35 (brs, 1H), 5.56 (brs, 2H), 3.77 (d, J=7.7 Hz, 2H), 3.34 (s, 3H), 2.76 (t, 1H), 2.40 - 1.50 (m, 13H), 0.99 (d, J=6.6 Hz, 6H)

The substantially same cyclization reaction as in Example 1 was performed except for using 2.60g (7.21 mmol) of the thus obtained compound to obtain 1.69g (yield: 68%) of the isolated Compound 30 as white needle crystals.
Melting point: 266.0 - 268.7°C (recrystallized from ethanol/water)

| Elementary analysis: $C_{19}H_{26}N_4O_2$ | | | |
|---|---|---|---|
| Calculated (%): | C 66.64, | H 7.65, | N 16.36 |
| Found (%) : | C 66.89, | H 7.44, | N 16.42 |

IR (KBr) νmax ($cm^{-1}$): 1708, 1652, 1495.
NMR ($CDC\ell_3$, 90 MHz) δ (ppm): 11.72 (brs, 1H), 3.98 (d, J=7.5 Hz, 2H), 3.44 (s, 3H), 2.77 (t, 1H), 2.52 - 1.60 (m, 13H), 0.95 (d, J=6.6 Hz, 6H).

Example 26

8-(Noradamantan-3-yl)-1,3-dipropyl-2-thioxanthine (Compound 31)

The substantially same operations as in Example 10 were repeated except for using 3.00g (12.4 mmol) of 5,6-diamino-1,3-dipropyl-2-thiouracil and 2.27g (13.6 mmol) of noradamantane-3-carboxylic acid to obtain 2.95g (yield: 60%) of amorphous 6-amino-5-(noradamantane-3-carbonylamino)-1,3-dipropyl-2-thiouracil.
NMR ($CDC\ell_3$, 90 MHz) δ (ppm): 7.50 (brs, 1H), 5.80 (brs, 2H), 4.60 - 4.25 (m, 4H), 2.72 (t, 1H), 2.40 - 1.50 (m, 16H), 1.20 - 0.80 (m, 6H).

The substantially same cyclization reaction as in Example 29 was performed except for using 2.70g (6.92 mmol) of the thus obtained compound instead of 6-amino-5-(adamantane-1-carbonylamino)-1,3-dipropyluracil to obtain 765 mg (yield: 30%) of the isolated Compound 31 as a light yellow powder.
Melting point: 216.2 - 216.6°C (recrystallized from isopropanol)

| Elementary analysis: $C_{20}H_{28}N_4OS$ | | | |
|---|---|---|---|
| Calculated (%): | C 64.48, | H 7.58, | N 15.04 |
| Found (%) : | C 64.49, | H 7.56, | N 15.35 |

IR (KBr) νmax ($cm^{-1}$): 1690, 1494.
NMR ($CDC\ell_3$) δ (ppm): 11.96 (brs, 1H), 4.69 (t, 2H), 4.61 (t, 2H), 2.86 (t, 1H), 2.48 - 2.42 (m, 2H), 2.35 - 2.26 (m, 2H), 2.15 - 1.85 (m, 12H), 1.15 - 0.95 (m, 6H).

Example 27

8-(Noradamantan-3-yl)-1,3-dipropyl-6-thioxanthine (Compound 32)

The substantially same operations as in Example 21 were repeated except for using 2.00g (5.62 mmol) of 8-(noradamantan-3-yl)-1,3-dipropylxanthine (Compound 14) prepared in Example 10 to obtain 2.02g (yield: 70%) of the isolated Compound 32 as light yellow crystals.
Melting point: 128.5 - 130.4°C (recrystallized from acetonitrile)

| Elementary analysis: $C_{20}H_{28}N_4OS$ | | | |
|---|---|---|---|
| Calculated (%): | C 64.48, | H 7.58, | N 15.04 |
| Found (%) : | C 64.49, | H 7.66, | N 15.29 |

IR (KBr) νmax ($cm^{-1}$): 1,682, 1,597 and 1,495
NMR ($CDC\ell_3$, 90 MHz) δ (ppm): 9.65 (brs, 1H), 4.43 (t, 2H), 4.06 (t, 2H), 2.69 (t, 1H), 2.53 - 1.60 (m, 16H), 1.10 - 0.85 (m, 6H).

Example 28

8-(Noradamantan-3-yl)-1,3-dipropyl-2,6-dithioxanthine (Compound 33)

The substantially same operations as in Example 21 were repeated except for using 2.00g (5.38 mmol) of 8-noradamantan-3-yl)-1,3-dipropyl-2-thioxanthine (Compound 31) prepared in Example 26 to obtain 1.27g (yield: 61%) of the isolated Compound 33 as a light yellow powder.
Melting point: 94.2 - 96.6°C (recrystallized from acetonitrile)

| Elementary analysis: $C_{20}H_{28}N_4S_2 \cdot 0.1CH_3CN \cdot 0.2H_2O$ | | | |
|---|---|---|---|
| Calculated (%): | C 61.22, | H 7.30, | N 14.49 |
| Found (%) : | C 61.18, | H 7.38, | N 14.57 |

IR (KBr) νmax ($cm^{-1}$): 1604, 1504, 1088.
NMR ($CDC\ell_3$, 90 MHz) δ (ppm): 9.46 (brs, 1H), 5.06 (t, 2H), 4.62 (t, 2H), 2.72 (t, 1H), 2.53 - 1.55 (m, 16H), 1.15 - 0.85 (m, 6H).

Example 29

8-(Adamantan-1-yl)-1,3-dipropylxanthine (Compound 13)

At first, 10g (44.3 mmol) of 1,3-dipropyl-5,6-diaminouracil were dissolved in 50 mℓ of pyridine, and 10.6 g (53.1 mmol) of adamantane-l-carbonylchloride were added by portions thereto at 0°C. After stirring for 30 minutes at 0°C, the mixture was concentrated under reduced pressure. A saturated aqueous sodium bicarbonate solution was added thereto. The residue was extracted with chloroform three times. The organic layers were combined, washed with saturated aqueous sodium chloride, and dried over anhydrous sodium sulfate. The solvent was evaporated under reduced pressure. Then pyridine was removed from the residue by means of azeotropic destillation with toluene to obtain 19.5 g (yield: 100%) of 6-amino-5-(adamantane-1-carbonylamino)-1,3-dipropyluracil.
NMR (90MHz; $CDC\ell_3$) δ (ppm): 7.47 (brs, 1H), 5.60 (brs, 2H), 4.05 - 3.70 (m, 4H), 2.25 - 1.45 (m, 19H), 1.15 - 0.85 (m, 6H)

Then, 100 ml of phosphorus oxychloride were added to 19.5 g of the thus obtained compound and the mixture was refluxed under heating for 30 minutes. The mixture was concentrated under reduced pressure, and a saturated aqueous sodium bicarbonate solution was added to the residue. The residue was extracted with chloroform three times. The extract was dried over anhydrous sodium sulfate and then the solvent was evaporated under reduced pressure. The residue was subjected to silica gel column chromatography (eluent: 20% ethyl acetate/hexane) and recrystallized from isopropanol-water to obtain 2.07 g (overall yield: 13%) of the isolated Compound 13 as white needle crystals.
Melting point: 169.3 - 171.0°C

| Elementary analysis: $C_{21}H_{30}N_4O_2$ | | | |
|---|---|---|---|
| Calculated (%): | C 68.08, | H 8.16, | N 15.12 |
| Found (%) : | C 68.10, | H 8.30, | N 15.09 |

IR (KBr) νmax ($cm^{-1}$): 1699, 1650, 1491
NMR ($CDC\ell_3$) δ (ppm): 11.70 (brs, 1H), 4.15 - 3.95 (m, 4H), 2.15 - 2.05 (m, 9H), 1.85 - 1.50 (m, 10H), 1.05 - 0.85 (m, 6H)

Example 30

8-(Adamantan-1-yl)-1,3-dipropyl-7-methylxanthine (Compound 19)

The substantially same operations as in Example 13 were repeated except for using 2.50 g (6.76 mmol) of 8-(adamantan-1-yl)-1,3-dipropylxanthine (Compound 13) prepared in Example 29 to obtain 2.16g (yield: 83%) of the isolated Compound 19 as white crystals.
Melting point: 79.8 - 80.9°C (recrystallized from ethanol/water)

| Elementary analysis: $C_{22}H_{32}N_4O_2$ | | | |
|---|---|---|---|
| Calculated (%): | C 68.17, | H 8.38, | N 14.57 |
| Found (%) : | C 68.28, | H 8.47, | N 14.72 |

IR (KBr) νmax ($cm^{-1}$): 1698, 1659.
NMR (DMSO-$d_6$) δ (ppm): 4.10 (s, 3H), 3.93 (t, 2H), 3.82 (t, 2H), 2.13 - 2.02 (m, 9H), 1.82 - 1.46 (m, 10H), 0.90 - 0.80 (m, 6H).

Pharmaceutical preparation 1

Tablet:

A tablet having the following composition was prepared according to a conventional method.

| | |
|---|---|
| Compound 3 | 20 mg |
| Lactose | 60 mg |
| Potato starch | 30 mg |
| Polyvinyl alcohol | 3 mg |
| Magnesium stearate | 1 mg |

Pharmaceutical preparation 2

Powder:

A powder having the following composition was prepared according to a conventional method.

| | |
|---|---|
| Compound 1 | 20 mg |
| Lactose | 300 mg |

Pharmaceutical preparation 3

Syrup:

A syrup having the following composition was prepared according to a conventional method.

| | |
|---|---|
| Compound 2 | 20 mg |
| Refined saccharose | 30 mg |

(continued)

| | |
|---|---|
| Ethyl p-hydroxybenzoate | 40 mg |
| Propyl p-hydroxybenzoate | 10 mg |
| Strawberry flavor | 0.1 mℓ |
| Water to make the total volume | 100 mℓ |

Pharmaceutical preparation 4

Capsule:

Ingredients set forth below were admixed and charged into gelatin capsules in accordance with a conventional method to thereby prepare a capsule.

| | |
|---|---|
| Compound 3 | 20 mg |
| Lactose | 200 mg |
| Magnesium stearate | 5 mg |

## Claims

**1.** Xanthine compounds represented by the following formula (I):

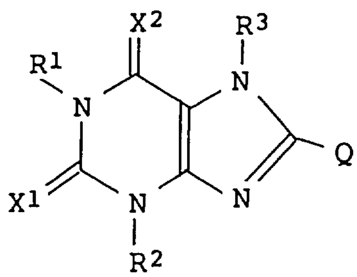

(I)

wherein each of $R^1$, $R^2$ and $R^3$ independently represents a hydrogen atom or a lower alkyl group having 1 to 6 carbon atoms; each of $X^1$ and $X^2$ independently represents an oxygen or sulfur atom; and Q represents:

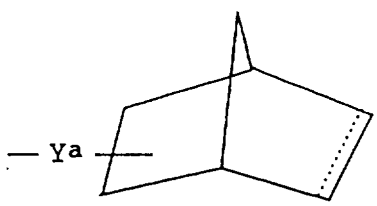
,
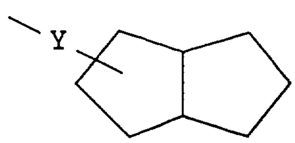
,
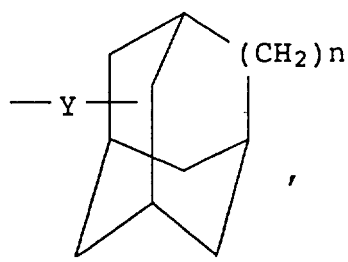
,

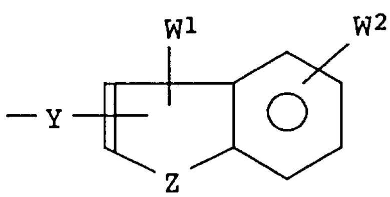
or
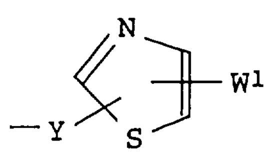

wherein ------ represents a single bond or a double bond;
$Y^a$ represents an alkylene group having 1 to 4 carbon atoms, Y represents a single bond or an alkylene group having 1 to 4 carbon atoms, n represents 0 or 1, each of $W^1$ and $W^2$ independently represents a hydrogen atom, a lower alkyl having 1 to 6 carbon atoms or amino group, Z represents $-CH_2-$, -O-, -S- or -NH- residues; provided that when Q is

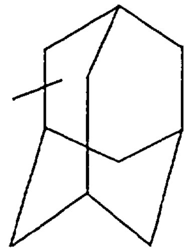,

then $R^1$, $R^2$ and $R^3$ are not simultaneously a methyl group;
or a physiologically acceptable salt thereof.

2. The compounds according to Claim 1, wherein both of $R^1$ and $R^2$ are lower alkyl groups having 1 to 6 carbon atoms and R3 is a hydrogen atom; and both of $X^1$ and $X^2$ are oxygen atoms.

3. The compounds according to Claim 1 or 2, wherein Q is

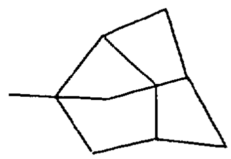.

4. The compounds according to Claim 1 or 2, wherein Q is

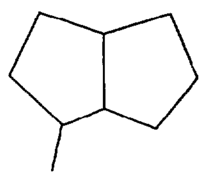.

5. The compounds according to Claim 1 or 2, wherein Q is

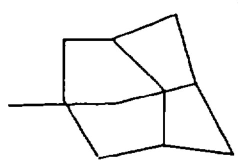.

6. 8-(Noradamantan-3-yl)-1,3-dipropylxanthine or a physiologically acceptable salt thereof.

7. Process for preparing xanthine compounds according to any of Claims 1 to 6,

a) by reacting a uracil derivative (II)

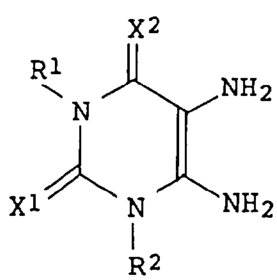

(II)

with a carboxylic acid (III)

$$Q-CO_2H \qquad (III)$$

without any solvent at a temperature of 50 to 200°C or with a carboxylic acid reactive derivative at a temperature of -80 to 50°C to obtain a compound (IV)

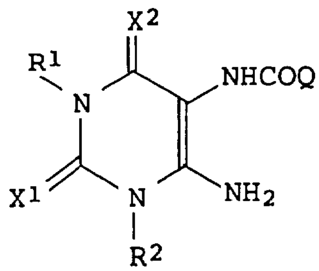

(IV)

which is further reacted

α) in the presence of a base
β) by treatment with a dehydrating agent or
γ) by heating to a compound of the general formula (I-1)

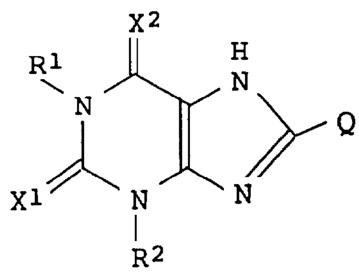

(I-1)

b) by reacting the compound of the general formula (II) with an aldehyde of the general formula (V)

$$Q\text{-}CHO \qquad (V)$$

in a mixed solvent at a temperature of -20 to 100°C to a Schiff base of the general formula (VI)

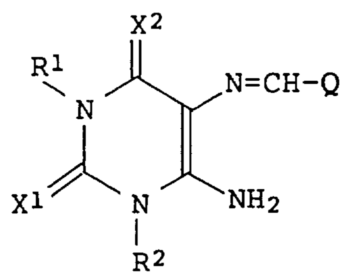

(VI)

which is further subjected to an oxidative cyclization reaction at a temperature from room temperature to 180°C in the presence of an oxidizing agent to obtain a compound of the general formula (I-1) or

c) by reacting a uracil derivative of the general formula (VII)

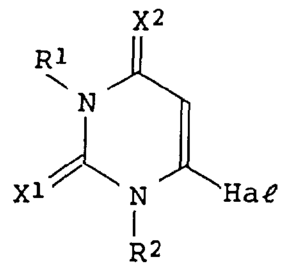

(VII)

wherein Hal represents halogen atoms with an amine of the general formula (VIII)

$$H_2NCH_2Q \qquad (VIII)$$

in an inert solvent at a temperature of 50 to 150°C to a compound of the general formula (IX)

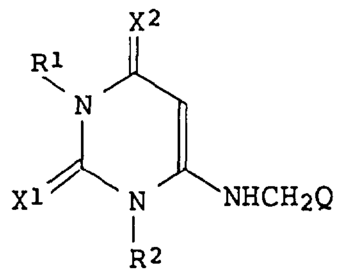

(IX)

which is further reacted with a nitrosating agent in an inert solvent at a temperature of room temperature to the boiling point of the solvent to obtain a compound of the general formula (I-1) whereby the compound of the general formula (I-1) optionally can be further alkylated by treatment with an alkylating agent to a compound of the general formula (I-2)

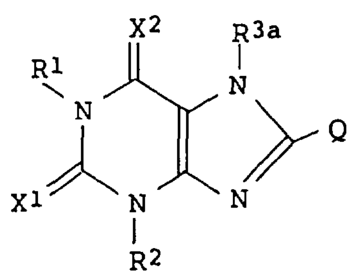

(I-2)

wherein $R^{3a}$ represents a lower alkyl group within the definition of $R^3$.

**8.** Use of the compounds of any of claims 1 to 6 for the preparation of a pharmaceutical composition.

**9.** A pharmaceutical composition comprising a pharmacologically acceptable carrier and, as an active ingredient, an effective amount of the xanthine compound as defined by any of Claims 1 to 6 or a physiologically acceptable salt thereof.

**10.** A method for the preparation of a pharmaceutical composition as defined in claim 9 comprising combining an effective amount of the xanthine compound as defined by any one of claims 1 to 6 or a pharmaceutically acceptable salt thereof with a pharmaceutically acceptable carrier.

**Patentansprüche**

**1.** Xanthinverbindungen der folgenden Formel (I):

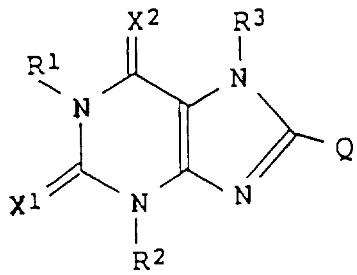

(I)

wobei jeder der Reste $R^1$, $R^2$ und $R^3$ unabhängig voneinander ein Wasserstoffatom oder einen Niederalkylrest mit 1 bis 6 Kohlenstoffatomen darstellt, jeder der Reste $X^1$ und $X^2$ unabhängig voneinander ein Sauerstoff- oder Schwefelatom bedeutet; und Q:

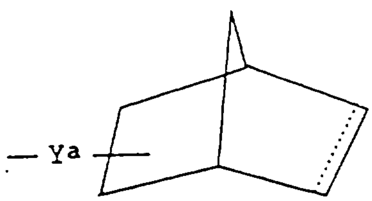

,

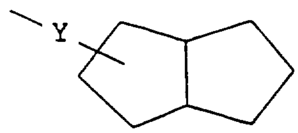

,

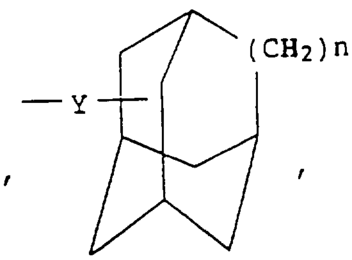

,

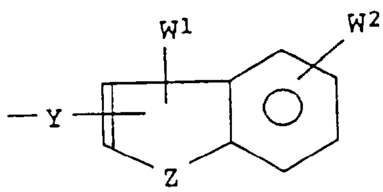

oder

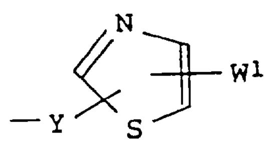

darstellt, wobei ----- eine Einfachbindung oder eine Doppelbindung wiedergibt, $Y^a$ einen Alkylenrest mit 1 bis 4 Kohlenstoffatomen bedeutet, Y eine Einfachbindung oder einen Alkylenrest mit 1 bis 4 Kohlenstoffatomen darstellt, n 0 oder 1 bedeutet, jeder der Reste $W^1$ und $W^2$ unabhängig voneinander ein Wasserstoffatom, einen Niederalkylrest mit 1 bis 6 Kohlenstoffatomen oder eine Aminogruppe darstellt, Z die Reste $-CH_2-$, -O-, -S- oder -NH- wiedergibt; mit der Maßgabe, daß $R^1$, $R^2$ und $R^3$ nicht gleichzeitig eine Methylgruppe bedeuten,wenn Q

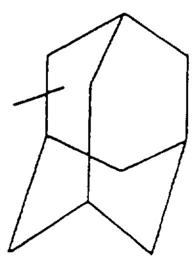

darstellt;
oder ein physiologisch verträgliches Salz davon.

2. Verbindungen nach Anspruch 1, wobei beide Reste $R^1$ und $R^2$ Niederalkylreste mit 1 bis 6 Kohlenstoffatomen darstellen und $R^3$ ein Wasserstoffatom bedeutet und beide Reste $X^1$ und $X^2$ Sauerstoffatome darstellen.

3. Verbindungen nach Anspruch 1 oder 2, wobei Q

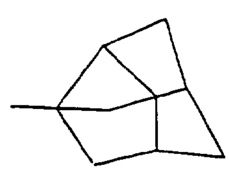

bedeutet.

**4.** Verbindungen nach Anspruch 1 oder 2, wobei Q

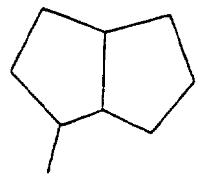

bedeutet.

**5.** Verbindungen nach Anspruch 1 oder 2, wobei Q

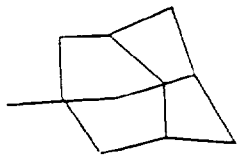

bedeutet.

**6.** 8-(Noradamantan-3-yl)-1,3-dipropylxanthin oder ein physiologisch verträgliches Salz davon.

**7.** Verfahren zur Herstellung von Xanthinverbindungen nach einem der Patentansprüche 1 bis 6,

a) durch Umsetzen eines Uracilderivats (II)

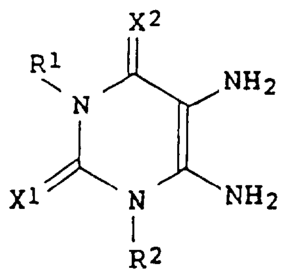

(II)

mit einer Carbonsäure (III)

$$Q - CO_2H \qquad (III)$$

ohne Lösungsmittel bei einer Temperatur von 50 bis 200°C oder mit einem reaktionsfähigen Carbonsäurederivat bei einer Temperatur von -80 bis 50°C, wobei eine Verbindung (IV)

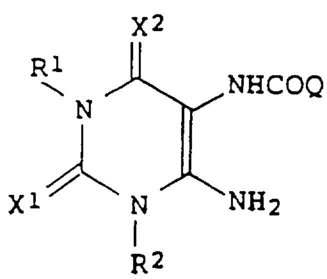

(IV)

erhalten wird, die weiter umgesetzt wird

α) in Gegenwart einer Base
β) durch Behandlung mit einem Dehydratisierungsmittel oder
γ) durch Erhitzen

zu einer Verbindung der allgemeinen Formel (I-1)

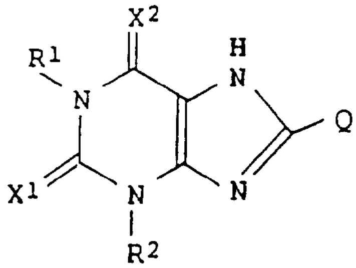

(I-1)

b) durch Umsetzen der Verbindung der allgemeinen Formel (II) mit einem Aldehyd der allgemeinen Formel (V)

Q -CHO (V)

in einem gemischten Lösungsmittel bei einer Temperatur von -20 bis 100°C zu einer Schiffschen Base der allgemeinen Formel (VI)

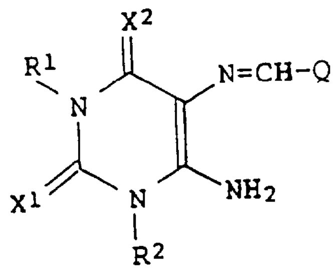

(VI)

die weiter einer oxidativen Cyclisierungsreaktion bei einer Temperatur von Raumtemperatur bis 180°C in Gegenwart eines Oxidationsmittels unterworfen wird, wobei eine Verbindung der allgemeinen Formel (I-1) erhalten wird oder

c) durch Umsetzen eines Uracilderivats der allgemeinen Formel (VII)

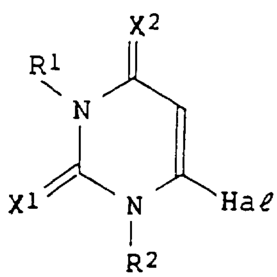

(VII)

wobei Hal Halogenatome darstellt, mit einem Amin der allgemeinen Formel (VIII)

$H_2NCH_2Q$ (VIII)

in einem inerten Lösungsmittel bei einer Temperatur von 50 bis 150°C zu einer Verbindung der allgemeinen Formel (IX)

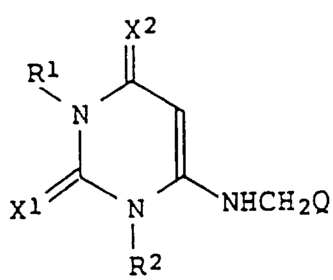

(IX)

die weiter mit einem Nitrosierungsmittel in einem inerten Lösungsmittel bei einer Temperatur von Raumtemperatur bis zum Siedepunkt des Lösungsmittels umgesetzt wird, wobei eine Verbindung der allgemeinen Formel (I-1) erhalten wird, wobei die Verbindung der allgemeinen Formel (I-1) gegebenenfalls durch Behandlung mit einem Alkylierungsmittel zu einer Verbindung der allgemeinen Formel (I-2)

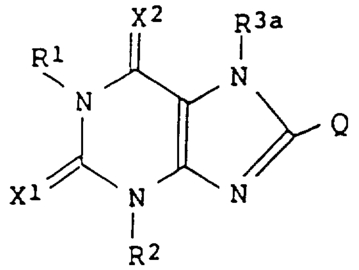

(I-2)

weiter alkyliert werden kann, in der $R^{3a}$ einen Niederalkylrest innerhalb der Definition von $R^3$ wiedergibt.

**8.** Verwendung der Verbindungen nach einem der Patentansprüche 1 bis 6 zur Herstellung eines Arzneimittels.

**9.** Arzneimittel, das einen pharmakologisch verträglichen Träger und als Wirkstoff eine wirksame Menge der Xanthinverbindung nach einem der Patentansprüche 1 bis 6 oder eines physiologisch verträglichen Salzes davon umfaßt.

**10.** Verfahren zur Herstellung eines Arzneimittels nach Anspruch 9, das das Kombinieren einer wirksamen Menge der Xanthinverbindung nach einem der Ansprüche 1 bis 6 oder eines pharmazeutisch verträglichen Salzes davon mit einem pharmazeutisch verträglichen Träger umfaßt.

## Revendications

**1.** Composés dérivés de la xanthine, représentés par la formule suivante (I) :

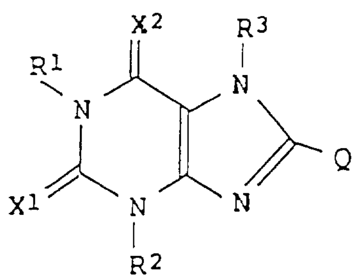

(I)

dans laquelle $R^1$, $R^2$ et $R^3$ représentent chacun, indépendamment, un atome d'hydrogène ou un groupe alkyle inférieur comportant de 1 à 6 atomes de carbone, $X^1$ et $X^2$ représentent chacun, indépendamment, un atome d'oxygène ou de soufre, et Q représente un reste de formule

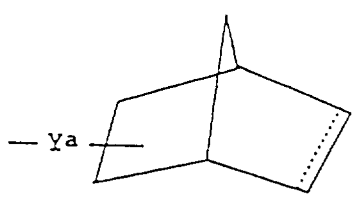

,

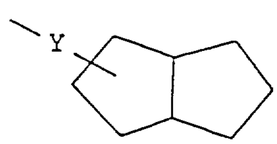

,

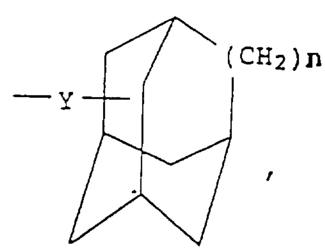

,

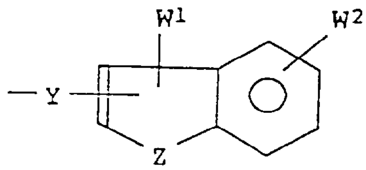

ou

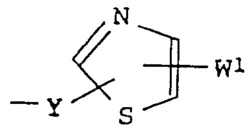

où ⎓ représente une liaison simple ou double, $Y^a$ représente un groupe alkylène comportant de 1 à 4 atomes de carbone, Y représente une liaison ou un groupe alkylène comportant de 1 à 4 atomes de carbone, n représente le nombre 0 ou 1, $W^1$ et $W^2$ représentent chacun, indépendamment, un atome d'hydrogène, un groupe alkyle inférieur comportant de 1 à 6 atomes de carbone ou un groupe amino, et Z représente $-CH_2-$, -O-, -S- ou -NH-, sous réserve que, si Q représente un reste de formule

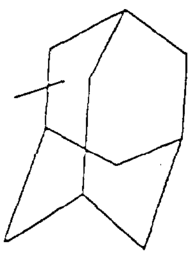

,

$R^1$, $R^2$ et $R^3$ ne peuvent pas représenter en même temps des groupes méthyle,
ou sel d'un tel dérivé, admissible du point de vue de la physiologie.

**2.** Composés conformes à la revendication 1, dans lesquels $R^1$ et $R^2$ représentent tous les deux des groupes alkyle inférieur comportant de 1 à 6 atomes de carbone et $R^3$ représente un atome d'hydrogène; et $X^1$ et $X^2$ représentent tous les deux des atomes d'oxygène.

**3.** Composés conformes à la revendication 1 ou 2, dans lesquels Q représente un reste de formule

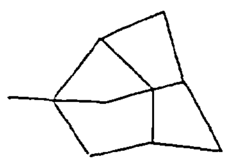

.

**4.** Composés conformes à la revendication 1 ou 2, dans lesquels Q représente un reste de formule

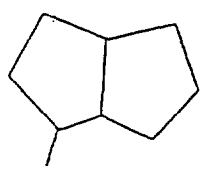

.

**5.** Composés conformes à la revendication 1 ou 2, dans lesquels Q représente un reste de formule

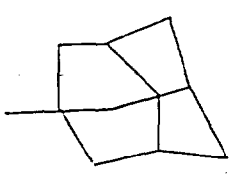

.

**6.** 8-(noradamantane-3-yle)-1,3-dipropyl-xanthine, ou l'un de ses sels admissibles du point de vue de la physiologie.

**7.** Procédé de préparation de dérivés de xanthine conformes à l'une des revendications 1 à 6, dans lequel :

a) on fait réagir un dérivé d'uracile de formule (II)

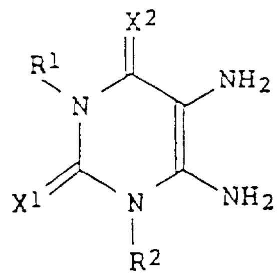

(II)

avec un acide carboxylique de formule (III)

$$Q\text{-}CO_2H \qquad \text{(III)}$$

en l'absence de tout solvant, à une température de 50°C à 200°C, ou bien avec un dérivé réactif d'acide carboxylique, à une température de -80°C à 50°C, ce qui donne un composé de formule (IV)

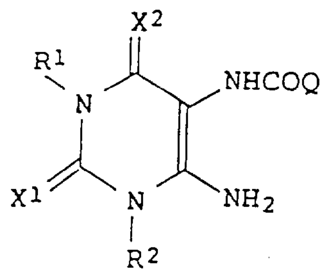

(IV)

que l'on fait ensuite réagir

α) en présence d'une base,
β) par traitement avec un agent déshydratant, ou encore
γ) par chauffage,

pour obtenir un composé de formule générale (I-1)

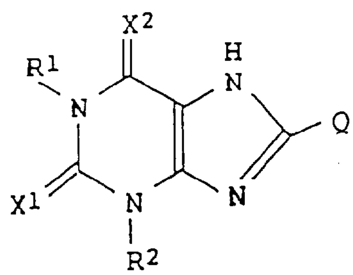

(I-1)

b) on fait réagir un composé de formule générale (II) avec un aldéhyde de formule générale (V)

$$Q\text{-}CHO \qquad \text{(V)}$$

dans un solvant mixte, à une température de -20°C à 100°C, ce qui donne une base de Schiff de formule générale (VI)

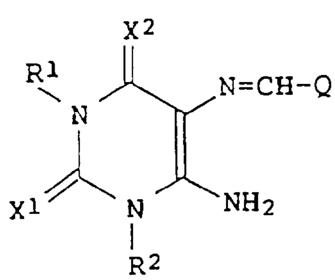

(VI)

que l'on soumet ensuite à une réaction de cyclisation oxydante, à une température située entre la température ambiante et 180°C et en présence d'un agent oxydant, pour obtenir un composé de formule générale (I-1),

ou bien
c) on fait réagir un dérivé d'uracile de formule générale (VII)

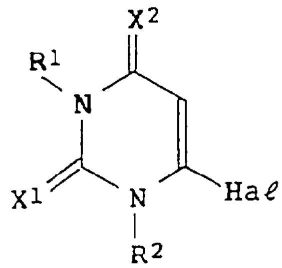

(VII)

dans laquelle Hal représente un atome d'halogène, avec une amine de formule générale (VIII)

$$H_2NCH_2Q \qquad (VIII)$$

dans un solvant inerte et à une température de 50°C à 150°C, ce qui donne un composé de formule générale (IX)

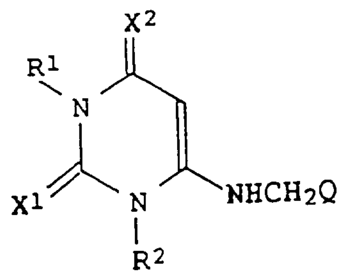

(IX)

que l'on fait ensuite réagir avec un agent de nitrosation, dans un solvant inerte et à une température située entre la température ambiante et le point d'ébullition du solvant, pour obtenir un composé de formule générale (I-1),
et l'on peut ensuite, si on le souhaite, alkyler le composé de formule générale (I-1) en le traitant avec un agent d'alkylation, pour obtenir un composé de formule générale (I-2)

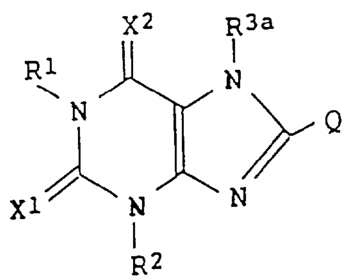

(I-2)

dans laquelle $R^{3a}$ représente un groupe alkyle inférieur qui entre dans le cadre de la définition donnée pour $R^3$.

**8.** Utilisation de l'un des composés conformes à l'une des revendications 1 à 6, dans la préparation d'une composition pharmaceutique.

**9.** Composition pharmaceutique comprenant un véhicule admissible en pharmacie et, comme ingrédient actif, une quantité efficace d'un dérivé de xanthine conforme à l'une des revendications 1 à 6 ou d'un sel d'un tel dérivé, admissible du point de vue de la physiologie.

**10.** Procédé de préparation d'une composition pharmaceutique conforme à la revendication 9, qui comporte le fait de combiner une quantité efficace d'un dérivé de xanthine conforme à l'une des revendications 1 à 6 ou d'un sel d'un tel dérivé, admissible en pharmacie, avec un véhicule admissible en pharmacie.